(19) Europäisches Patentamt
European Patent Office
Office européen des brevets

(11) **EP 4 603 015 A1**

(12) **EUROPEAN PATENT APPLICATION**
published in accordance with Art. 153(4) EPC

(43) Date of publication:
**20.08.2025   Bulletin 2025/34**

(21) Application number: **23877075.4**

(22) Date of filing: **14.09.2023**

(51) International Patent Classification (IPC):
*A61B 5/12* *(2006.01)*

(52) Cooperative Patent Classification (CPC):
**A61B 5/12**

(86) International application number:
**PCT/JP2023/033500**

(87) International publication number:
**WO 2024/080069 (18.04.2024 Gazette 2024/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **11.10.2022   JP 2022163328**

(71) Applicant: **Sony Group Corporation**
**Tokyo 108-0075 (JP)**

(72) Inventors:
• **NAKAMURA, Osamu**
  **Tokyo 108-0075 (JP)**
• **MATSUMOTO, Kyosuke**
  **Tokyo 108-0075 (JP)**
• **MAKINO, Kenichi**
  **Tokyo 108-0075 (JP)**
• **TSUCHIYA, Shinpei**
  **Tokyo 108-0075 (JP)**

(74) Representative: **D Young & Co LLP**
**3 Noble Street**
**London EC2V 7BQ (GB)**

(54) **INFORMATION PROCESSING DEVICE, INFORMATION PROCESSING METHOD, AND PROGRAM**

(57)   There is provided an information processing device including a prediction unit that predicts a degree of a conductive hearing loss based on test results of an auditory test using air conduction sound, the auditory test being first and second auditory tests including test contents different from each other.

## FIG.12

EP 4 603 015 A1

**Description**

Field

The present disclosure relates to an information processing device, an information processing method, and a program.

Background

When considering the use of a hearing aid, a medical institution, a hearing aid store, or the like performs a pure sound audiometry first. The pure sound audiometry is a hearing test performed using test equipment called audiometer and, specifically, an air conduction hearing test and a bone conduction hearing test are performed using the audiometer. For example, the air conduction hearing test and the bone conduction hearing test are performed using a method described in Non Patent Literature 1 below. Then, a difference between an air conduction hearing level obtained by the air conduction hearing test and a bone conduction hearing level obtained by the bone conduction hearing test is calculated and a degree of a conductive hearing loss can be grasped. Further, information concerning the degree of the conductive hearing loss is used to determine, before starting wearing of a hearing aid, whether to preferentially perform study of treatment other than the hearing aid. In addition, the information concerning the degree of the conductive hearing loss is also used to determine a gain of the hearing aid.

Citation List

Non Patent Literature

Non Patent Literature 1: Hearing Test Method of Japanese Society of Hearing Medicine 1. Pure Sound Hearing (Threshold) Level Measurement Method by Audiometer (2008)

Summary

Technical Problem

When the bone conduction hearing test is performed, a bone conduction headset is worn on the head of a subject. However, since the bone conduction headset needs to be accurately attached to a place where test sound can be efficiently transmitted, it is difficult for a person other than an expert to attach the bone conduction headset. Therefore, for example, it is not easy for an individual subject to perform the bone conduction hearing test and obtain information concerning the degree of the conductive hearing loss.

Therefore, the present disclosure proposes an information processing device, an information processing method, and a program that can easily predict a degree of a conductive hearing loss using air conduction sound.

Solution to Problem

According to the present disclosure, there is provided an information processing device including a prediction unit that predicts a degree of a conductive hearing loss based on test results of an auditory test using air conduction sound. In the information processing device, the auditory test is first and second auditory tests including test contents different from each other.

Furthermore, according to the present disclosure, there is provided an information processing method including predicting, by an information processing device, a degree of a conductive hearing loss based on test results of an auditory test using air conduction sound. In the information processing method, the auditory test is first and second auditory tests including test contents different from each other.

Furthermore, according to the present disclosure, there is provided a program causing a computer to execute: a function of predicting a degree of a conductive hearing loss based on test results of an auditory test using air conduction sound. In the program, the auditory test is first and second auditory tests including test contents different from each other.

Brief Description of Drawings

FIG. 1 is an explanatory diagram for explaining a difference in a path between air conduction sound and bone conduction sound.

FIG. 2 is an explanatory diagram for explaining a corresponding range of an impaired site in pure sound audiometry.

FIG. 3 is a flowchart for explaining a flow of determination of necessity of treatment before wearing of a hearing aid.

FIG. 4 is an explanatory diagram for explaining a setting example of a hearing aid gain for each type of a hearing loss.

FIG. 5 is an explanatory diagram for explaining an example of a pure sound audiometry of the related art.

FIG. 6 is an explanatory diagram for explaining a hearing test according to an embodiment of the present disclosure.

FIG. 7 is an explanatory diagram for explaining an example of a hearing test of the related art.

FIG. 8 is an explanatory diagram for explaining an example of an observation result by a self-recording audiometer.

FIG. 9 is an explanatory diagram for explaining a correspondence range of the self-recording audiometry with respect to an impaired site.

FIG. 10 is an explanatory diagram for explaining an overview of a first embodiment of the present disclosure.

FIG. 11 is an explanatory diagram for explaining learning data according to the first embodiment of the present disclosure.

FIG. 12 is a block diagram of an information processing terminal according to the first embodiment of the present disclosure.

FIG. 13 is an explanatory diagram for explaining a conductive hearing loss prediction unit according to the first embodiment of the present disclosure.

FIG. 14 is a flowchart (part 1) for explaining a flow of an information processing method according to the first embodiment of the present disclosure.

FIG. 15 is a flowchart (part 2) for explaining the flow of the information processing method according to the first embodiment of the present disclosure.

FIG. 16 is an explanatory diagram for explaining an example of an auditory test service according to the first embodiment of the present disclosure.

FIG. 17 is an explanatory diagram for explaining an overview of a second embodiment of the present disclosure.

FIG. 18 is a block diagram of an information processing terminal according to the second embodiment of the present disclosure.

FIG. 19 is a flowchart for explaining a flow of an information processing method according to the second embodiment of the present disclosure.

FIG. 20 is a flowchart (part 1) for explaining a flow of an information processing method according to a third embodiment of the present disclosure.

FIG. 21 is a flowchart (part 1) for explaining a flow of an information processing method according to a modification of the third embodiment of the present disclosure.

FIG. 22 is a flowchart (part 2) for explaining the flow of the information processing method according to the modification of the third embodiment of the present disclosure.

FIG. 23 is a flowchart (part 2) for explaining the flow of the information processing method according to the third embodiment of the present disclosure.

FIG. 24 is a block diagram of an information processing terminal according to a fourth embodiment of the present disclosure.

FIG. 25 is a flowchart for explaining a flow of an information processing method according to the fourth embodiment of the present disclosure.

FIG. 26 is an explanatory diagram for explaining the fourth embodiment of the present disclosure.

FIG. 27 is an explanatory diagram (part 1) for explaining a display example according to the fourth embodiment of the present disclosure.

FIG. 28 is an explanatory diagram (part 2) for explaining a display example according to the fourth embodiment of the present disclosure.

FIG. 29 is an explanatory diagram (part 1) for explaining an application example according to the fourth embodiment of the present disclosure.

FIG. 30 is an explanatory diagram (part 2) for explaining an application example according to the fourth embodiment of the present disclosure.

FIG. 31 is a block diagram of an external device according to the fourth embodiment of the present disclosure.

FIG. 32 is a diagram illustrating a schematic configuration of a hearing aid system according to an embodiment of the present disclosure.

FIG. 33 is a functional block diagram of a hearing aid and a charger according to the embodiment of the present disclosure.

FIG. 34 is a block diagram of an information processing terminal according to the embodiment of the present disclosure.

FIG. 35 is a block diagram of a server according to the embodiment of the present disclosure.

FIG. 36 is a diagram illustrating an example of utilization of data.

FIG. 37 is a diagram illustrating an example of data.
FIG. 38 is a diagram illustrating an example of cooperation with another device.
FIG. 39 is a diagram illustrating an example of use transition.

Description of Embodiments

Preferred embodiments of the present disclosure are explained in detail below with reference to the accompanying drawings. Note that, in the present specification and the drawings, components having substantially the same functional configurations are denoted by the same reference numerals and signs, whereby redundant explanation of the components is omitted. In addition, in the present specification and the drawings, a plurality of components having substantially the same or similar functional configurations are sometimes distinguished by attaching different alphabets after the same reference numerals. However, when it is not particularly necessary to distinguish each of the plurality of components having substantially the same or similar functional configurations, only the same reference numerals and signs are attached.

Note that the explanation is made in the following order.

1. Background leading to creation of embodiments of present disclosure
2. First Embodiment
2.1 Overview
2.2 Configuration
2.3 Information processing method
2.4 Application example
3. Second Embodiment
3.1 Overview
3.2 Configuration
3.3 Information processing method
4. Third Embodiment
5. Fourth Embodiment
5.1 Configuration
5.2 Information processing method
5.3 Display example
5.4 Application example
6. Summary
7. Overview of hearing aid system
8. Example of utilization of data
9. Example of cooperation with other devices
10. Example of use transition
11. Supplement

<<1. Background leading to creation of embodiments of present disclosure>>

First, before embodiments of the present disclosure are explained, a background leading to creation of embodiments of the present disclosure by the present inventors is explained.

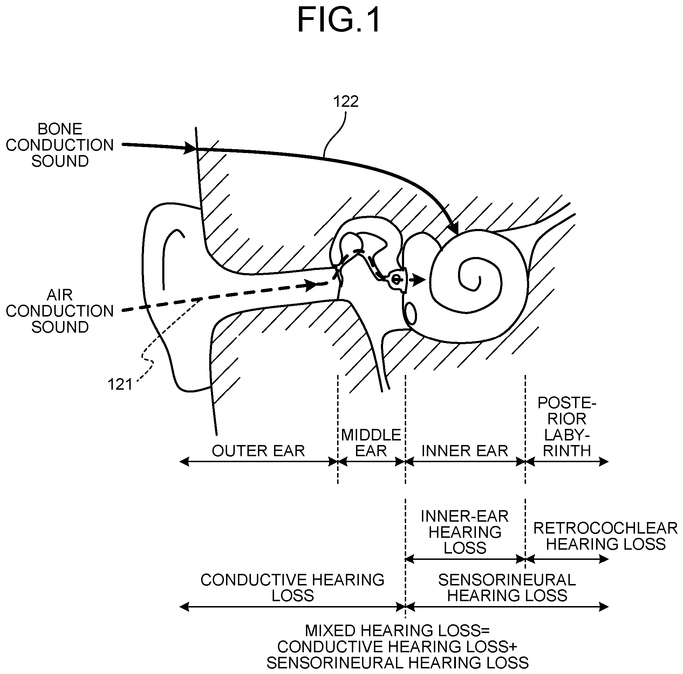

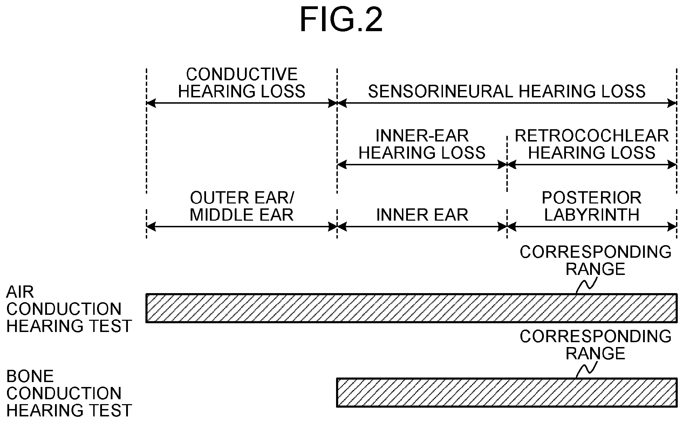

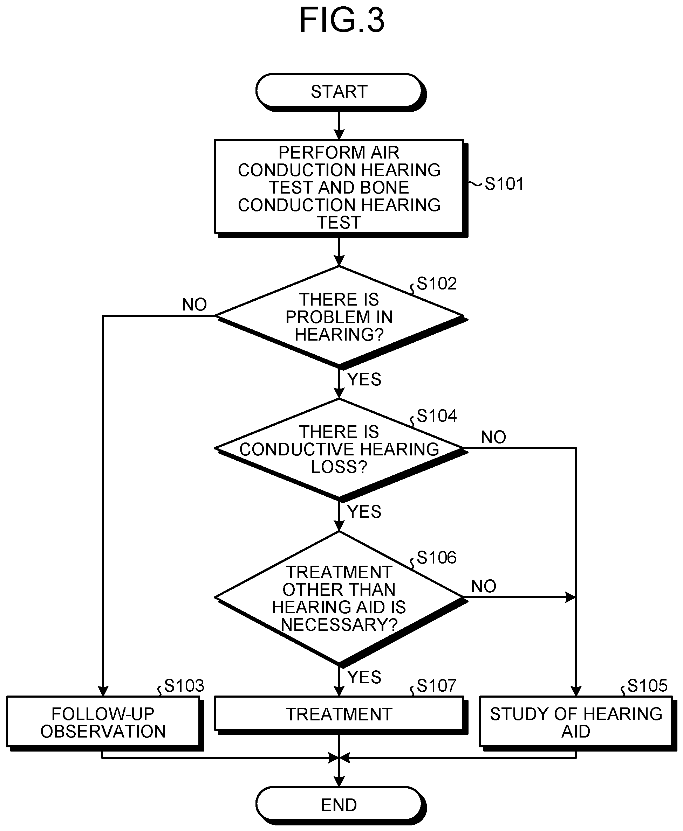

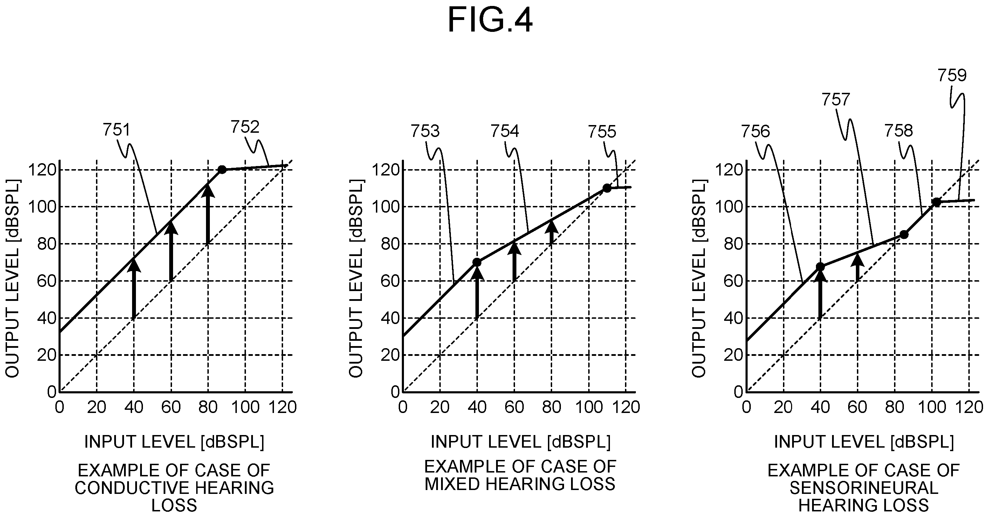

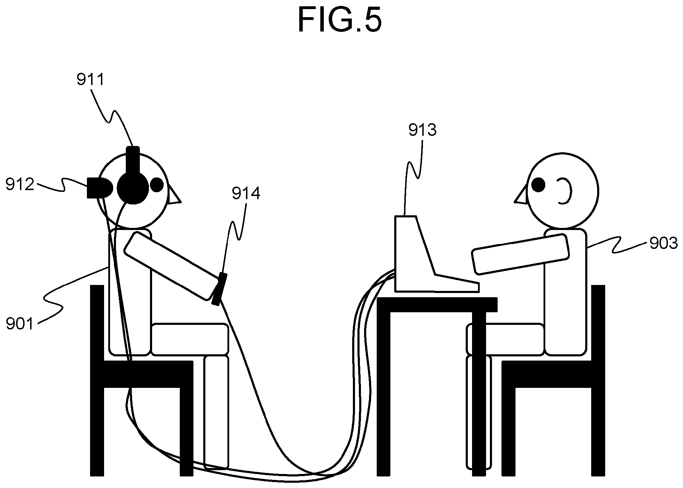

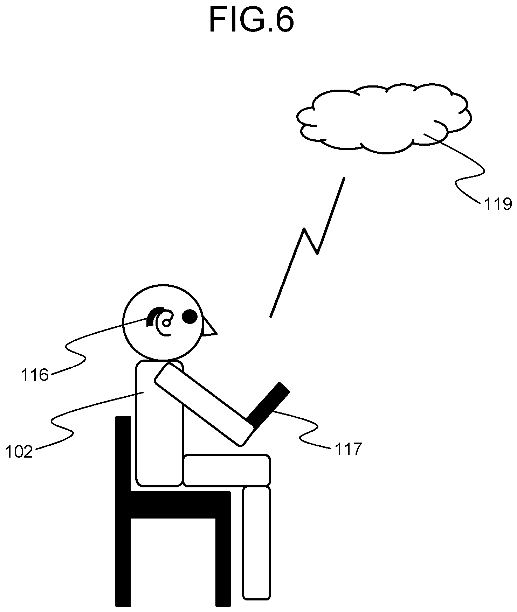

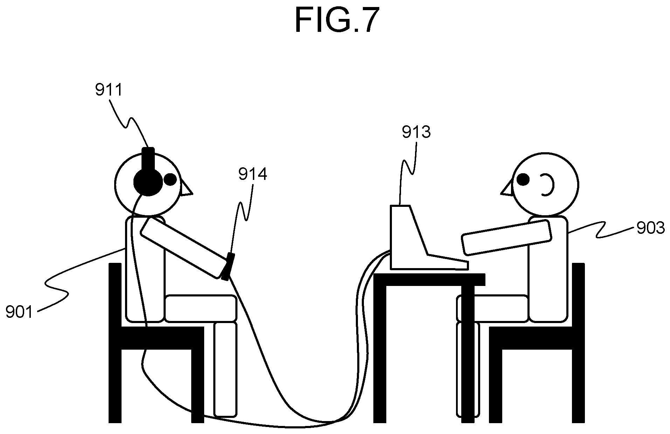

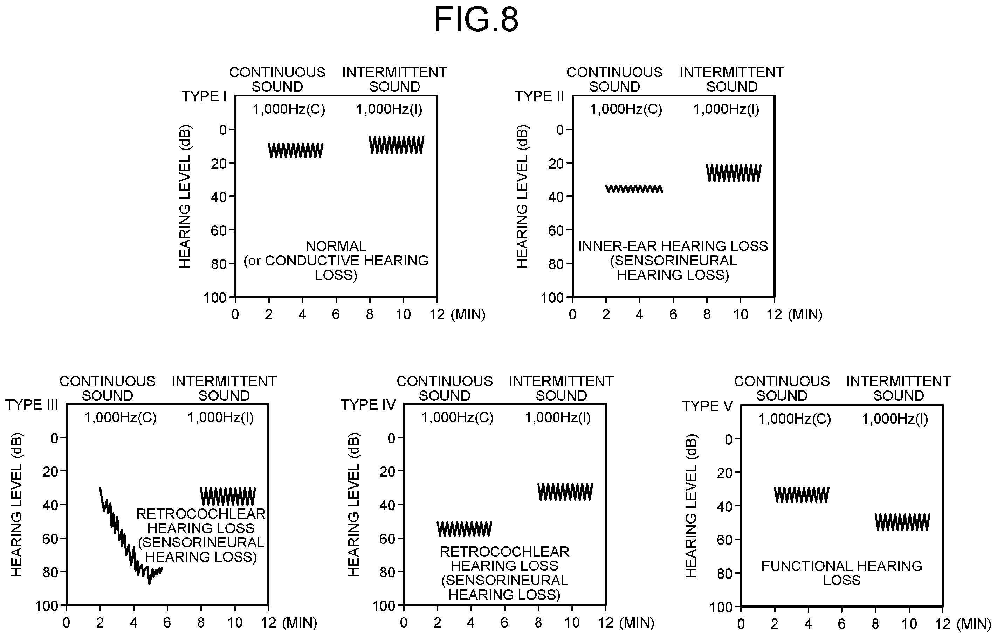

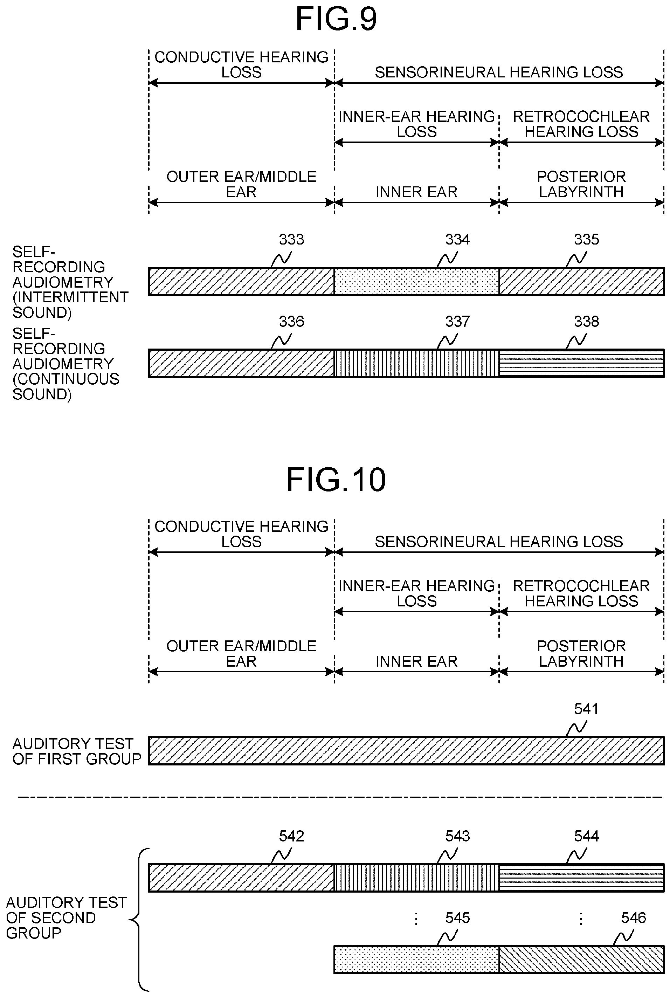

First, studies on wearing of a hearing aid are explained with reference to FIG. 1 to FIG. 9. FIG. 1 is an explanatory diagram for explaining a difference in path between air conduction sound and bone conduction sound, FIG. 2 is an explanatory diagram for explaining a corresponding range of an impaired site in a pure sound audiometry, and FIG. 3 is a flowchart for explaining a flow of determination on necessity of treatment before wearing of a hearing aid. FIG. 4 is an explanatory diagram for explaining a setting example of a hearing aid gain for each type of a hearing loss, FIG. 5 is an explanatory diagram for explaining an example of a pure sound audiometry of the related art, and FIG. 6 is an explanatory diagram for explaining a hearing test according to an embodiment of the present disclosure. Further FIG. 7 is an explanatory diagram for explaining an example of a hearing test of the related art, FIG. 8 is an explanatory diagram for explaining an example of an observation result by a self-recording audiometer, and FIG. 9 is an explanatory diagram for explaining a corresponding range of the self-recording audiometry with respect to an impaired site.

When considering wearing of a hearing aid, a medical institution, a hearing aid store, or the like performs a pure sound audiometry on a user (a subject). The pure sound audiometry includes two types of threshold tests of an air conduction hearing test and a bone conduction hearing test. Usually, both of the tests are performed.

In FIG. 1, a first path 121 indicates a path of air conduction sound. The air conduction sound is sound that reaches an inner ear as vibration after passing through an outer ear and a middle ear. The vibration is then converted into an electric signal in the inner ear. Further, the electric signal travels through a posterior labyrinthine towards an auditory cortex. Then, the air conduction hearing test is a test in which test sound is delivered to the inner ear using the first path 121. In the air conduction hearing test, since the air conduction sound reaches the inner ear via the outer ear and the middle ear, it is possible to measure a state in which degrees of disorders of the outer ear, the middle ear, the inner ear, and the posterior labyrinthine are added up. A hearing loss due to disorders of the outer ear and the middle ear is referred to as conductive hearing loss, a hearing loss due to a disorder of the inner ear is referred to as inner-ear hearing loss, a hearing loss due to a disorder of the posterior labyrinthine is referred to as retrocochlear hearing loss, and the inner-ear hearing loss and the retrocochlear hearing loss are collectively referred to as sensorineural hearing loss. In addition, hearing loss in which conductive hearing loss and sensorineural hearing loss occur simultaneously is referred to as mixed hearing loss. That is, the air conduction hearing test can also be considered a test that can measure a degree of the conductive hearing loss and a degree of the sensorineural hearing loss in an added-up state. A hearing level measured in the air conduction hearing test is referred to as air conduction hearing level. The air conduction hearing level can be represented by the following formula (1).

$$\text{Air conduction hearing level} = \text{degree of conductive hearing loss} + \text{degree of sensorineural hearing loss} \quad \cdots (1)$$

On the other hand, in FIG. 1, a second path 122 indicates a path of bone conduction sound. The bone conduction sound is sound that reaches the inner ear as vibration after passing through a skull. After reaching the inner ear, the bone conduction sound is the same as the case of the air conduction sound explained above. The bone conduction hearing test is a test in which test sound is delivered to the inner ear using the second path 122. In the bone conduction hearing test, since the bone conduction sound reaches the inner ear without passing through the outer ear and the middle ear, it is possible to measure the added-up state of the degrees of the disorders of the inner ear and the posterior labyrinth without being affected by the disorders of the outer ear and the middle ear. That is, the bone conduction hearing test can also be considered a test that can measure the degree of the sensorineural hearing loss without being affected by the conductive hearing loss. The hearing level measured in the bone conduction hearing test is referred to as bone conduction hearing level. The bone conduction hearing level can be represented by the following formula (2).

$$\text{Bone conduction hearing level} = \text{degree of sensorineural hearing loss} \quad \cdots (2)$$

Further, a difference between the air conduction hearing level and the bone conduction hearing level is referred to as air bone gap. The air bone gap represents the degree of conductive hearing loss and can be represented by the following formula (3).

$$\text{Air bone gap} = \text{air conduction hearing level} - \text{bone conduction hearing level}$$
$$= \text{degree of conductive hearing loss} \quad \cdots (3)$$

FIG. 2 illustrates a corresponding range of the air conduction hearing test and the bone conduction hearing test of the pure sound audiometry for an impaired site. The air conduction hearing test can be used to obtain indicators of disorders causing the conductive hearing loss, the inner-ear hearing loss, and the retrocochlear hearing loss. The bone conduction hearing test can be used to obtain indicators of disorders causing the inner-ear hearing loss and the retrocochlear hearing loss. Since the same test sound is used in both of the air conduction hearing test and the bone conduction hearing test, responses to the measurements at impaired sites are the same. For that reason, the air bone gap obtained by subtracting the bone conduction hearing level from the air conduction hearing level indicates a degree of the conductive hearing loss. Note that this is as explained using the formula (1) to the formula (3).

For example, when a disorder equivalent to 40 dBHL is present only in the inner ear (a sensorineural hearing loss of 40 dBHL), both of a result of the air conduction hearing test and a result of the bone conduction hearing test are approximately 40 dBHL. For example, when a disorder equivalent to 30 dBHL is present only in the posterior labyrinthine (a sensorineural hearing loss of 30 dBHL), both of a result of the air conduction hearing test and a result of the bone conduction hearing test are approximately 30 dBHL. In contrast, for example, when a disorder equivalent to 35 dBHL is present only in the middle ear (a conductive hearing loss of 35 dBHL), a result of the air conduction hearing test is approximately 35 dBHL but a result of the bone conduction hearing test is approximately 0 dBHL. For example, when a disorder equivalent to 30 dBHL is present in the middle ear and a disorder equivalent to 35 dBHL is present in the inner ear, a mixed hearing loss of 65 dBHL is caused (30 dBHL for a conductive hearing loss and 35 dBHL for a sensorineural hearing loss). In such a case, a result of the air conduction hearing test is approximately 65 dBHL and a result of the bone conduction hearing test is approximately 35 dBHL.

As explained above, the air conduction hearing test and the bone conduction hearing test are tests that can distinguish a conductive hearing loss and a sensorineural hearing loss and can measure degrees of the conductive hearing loss and the sensorineural hearing loss.

Then, information concerning the degree of the conductive hearing loss is important in two points when considering use of a hearing aid. As a first point, the information concerning the degree of the conductive hearing loss is used to determine whether study of treatment other than a hearing aid should be preferentially performed before wearing of the hearing aid is started. As a second point, the information concerning the degree of the conductive hearing loss and the information concerning the degree of the sensorineural hearing loss are used when a gain of the hearing aid is determined.

First, the first point is explained. When a conductive hearing loss is suspected, study of treatment other than a hearing aid should be preferentially performed before wearing of the hearing aid. If a disorder causing a conductive hearing loss occurs, it is highly likely that the disorder can be ameliorated by early treatment. Conversely, when time elapses, it is likely that the disorder cannot be ameliorated by the treatment. Thus, when a conductive hearing loss is suspected, it is desirable to visit a medical institution or the like first and determine the necessity of treatment that should be prioritized. When there is treatment that should be prioritized, the treatment is performed first. As explained above, when considering wearing of a hearing aid, it is important to grasp a degree of the conductive hearing loss. Note that, when there is no treatment that should be prioritized even if a conductive hearing loss is present, for example, when the conductive hearing loss remains although the conductive hearing loss has already been treated, wearing of a hearing aid is considered.

With reference to a flowchart illustrated in FIG. 3, an example of a flow of determination of necessity of treatment before wearing of a hearing aid is explained. First, an air conduction hearing test and a bone conduction hearing test are performed on a subject (step S101). Then, it is determined whether there is a problem in hearing of the subject (step S102). In step S102, for example, it is determined whether hearing aid wearing is at an appropriate level. Specifically, in Japan, a hearing level of 25 dBHL or more and less than 40 dBHL is defined as a mild hearing loss and a hearing level of 40 dBHL or more and less than 70 dBHL is defined as a moderate hearing loss. Further, in Japan, in general, a hearing aids is worn when the hearing level is 40 dBHL or more. However, a criterion of determination can vary depending on a situation. Then, when it is determined that there is no problem in the hearing of the subject (step S102: No), the processing proceeds to step S103. Subsequently, it is determined that follow-up observation is periodically performed on the subject according to necessity (step S103) and the processing is ended.

On the other hand, when it is determined that there is a problem in the hearing of the subject (step S102: Yes), the processing proceeds to step S104. Subsequently, it is determined whether there is a conductive hearing loss (step S104). Whether there is a conductive hearing loss can be determined by an air bone gap. From the viewpoint of treatment, for example, a criterion for determining that there is a conductive hearing loss when the air bone gap is 25 dBHL or more is widely used. Then, when it is determined that there is no conductive hearing loss (step S104: No), the processing proceeds to step S105. Subsequently, it is recommended to the subject to consider a hearing aid (step S105) and the processing is ended.

On the other hand, when it is determined that the subject has a conductive hearing loss (step S104: Yes), the processing proceeds to step S106. Subsequently, it is determined whether treatment other than the hearing aid is necessary (step S106). The determination is usually made in a medical institution or the like. When it is determined that treatment is unnecessary (step S106: No), the processing proceeds to step S105. On the other hand, when it is determined that treatment is necessary (step S106: Yes), the processing proceeds to step S107. Then, it is determined to perform treatment on the subject (step S107) and the processing is ended.

As it is seen from FIG. 3, depending on whether there is treatment that should be prioritized other than the hearing aid before wearing of the hearing aid, a future policy is greatly different as to whether to perform the treatment first or to consider wearing of the hearing aid. For that reason, in order to prevent the subject from missing an opportunity of treatment, in other words, in order to prevent the subject from missing an opportunity of improvement of a hearing loss, it is important to grasp a degree of a conductive hearing loss.

Subsequently, the second point is explained. FIG. 4 illustrates a setting example of a hearing aid gain for each type of a hearing loss. Specifically, an example of gain setting of a hearing aid in the case of a conductive hearing loss is illustrated on the left side of FIG. 4, an example of gain setting of the hearing aid in the case of a mixed hearing loss is illustrated in the center of FIG. 4, and an example of gain setting of the hearing aid in the case of a sensorineural hearing loss is illustrated on the right side of FIG. 4. The mixed hearing loss is a hearing loss in which the conductive hearing loss and the sensorineural hearing loss simultaneously occur.

In the case of the conductive hearing loss, since there is no disorder in the inner ear, it is considered desirable to amplify, with a constant gain, sound collected by a microphone of the hearing aid. Amplifying the sound with the constant gain regardless of an input level in this way is generally called linear amplification. In the example illustrated on the left side of FIG. 4, in a first section 751, the sound is amplified with the constant gain regardless of the input level. Specifically, an inclination of a straight line in the first section 751 is 45 degrees. Note that the inclination of the straight line is not limited to this. In the example illustrated on the left side of FIG. 4, a second section 752 is provided as an output restriction and is usually provided in order to prevent a hearing loss from being further deteriorated by strong sound.

Since most of sensorineural hearing loss has an inner-ear hearing loss, a replenishment phenomenon often appears. When the replenishment phenomenon occurs, it is desirable to amplify sound by increasing a gain for a weak input level and decreasing a gain for a strong input level. Amplifying the sound with different gains depending on the input levels is generally called non-linear amplification. In the example illustrated on the right side of FIG. 4, sound is amplified with different gains depending on input levels in a sixth section 756, a seventh section 757, and an eighth section 758. Specifically, an inclination of a straight line in the seventh section 757 is closer to horizontal than 45 degrees. A ninth section 759 in the example illustrated on the right side of FIG. 4 is provided as an output restriction.

Here, the replenishment phenomenon will be described. The replenishment phenomenon is also referred to as recruitment phenomenon and is loudness abnormality (sensation of magnitude of sound) caused by a disorder of outer hair cells present in the cochlea of the inner ear. When the replenishment phenomenon is present, since the subject is sensitive to even a small volume change, a symptom sometimes occurs in which, for example, although the subject less easily hears small sound, the subject feels large sound as extremely large.

In the case of the mixed hearing loss, for example, sound is set to be amplified with a gain between the conductive hearing loss and the sensorineural hearing loss. In the example illustrated in the center of FIG. 4, an inclination in a fourth section 754 is an inclination between the inclination in the first section 751 and the inclination in the seventh section 757. Note that a degree of the inclination in the fourth section 754 is determined by, for example, a degree of conductive hearing loss and a degree of sensorineural hearing loss. A fifth section 755 in the example illustrated in the center of FIG. 4 is provided as an output restriction.

As it is seen from FIG. 4, gain setting necessary for the hearing aid is greatly different between the conductive hearing loss and the sensorineural hearing loss. For this reason, in order to perform adjustment suitable for the hearing of the subject who is a user of the hearing aid, it is important to grasp the degree of the conductive hearing loss and the degree of the sensorineural hearing loss. Thus, when hearing aid wearing is considered, a hearing test is performed on the subject.

FIG. 5 illustrates an example of a pure sound audiometry of the related art. Equipment that performs the pure sound audiometry is generally called an audiometer. An audiometer 913 includes an air conduction headset 911, a bone conduction headset 912, and a response button 914. In a medical institution, a hearing aid store, or the like, the pure sound audiometry is performed using the audiometer 913.

When the air conduction hearing test included in the pure sound audiometry is performed, the air conduction headset 911 is worn on the head of a subject 901 to cause the subject 901 to hear test sound. Then, the test sound reaches the inner ear via the first path 121 illustrated in FIG. 1. When the test sound is heard, the subject 901 answers by pressing the response button 914. As the air conduction headset 911, there are an overhead type and an inner ear type. An air conduction headset of the overhead type has a form similar to a general overhead headphone for music appreciation. An air conduction headset of the inner ear type has a form similar to a general inner earphone for music appreciation.

When the bone conduction hearing test included in the pure sound audiometry is performed, the bone conduction headset 912 is worn on the head of the subject 901 to cause the subject 901 to hear test sound. The test sound reaches the inner ear via the second path 122 illustrated in FIG. 1. At this time, in principle, an ear opposite to an inspected ear (an ear on a side to be inspected) is masked with a not-illustrated air conduction headset for masking or the like. Since the bone conduction headset 912 vibrates the skull, test sound emitted from the skull is heard with both the ears at the same time. Therefore, unless masking is performed, a test for each ear cannot be performed. Therefore, the masking is performed to prevent the subject from hearing the test sound with the opposite ear and responding. When the test sound is heard, the subject 901 is presses the response button 914 to answer. An attachment position of the bone conduction headset 912 is often the mastoid (behind the auricle) or the frontal middle portion. The bone conduction headset 912 needs to be accurately worn on a place where the test sound can be efficiently transmitted such as the mastoid and the frontal middle portion. Therefore, attention is required when the bone conduction headset 912 is worn. Further, the bone conduction headset 912 must not touch the auricle. It is also inappropriate that the bone conduction headset 912 holds hair. In addition, since the air introduction receiver for masking (not illustrated) is also worn at the same time, it is also necessary to prevent the positions of the bone conduction headset 912 and the air conduction headset for masking from deviating. Therefore, it is preferable that the bone conduction headset 912 is worn on the head of the subject 901 by an expert 903.

FIG. 6 illustrates an example of a hearing test in the case in which a subject 102 purchases a hearing aid via the Internet or the like without visiting a medical institution, a hearing aid store, or the like. Hearing aids have been mainly sold via the hearing aid expert 903 working in a medical institution, a hearing aid store, or the like. On the other hand, in recent years, a form of selling hearing aids not via the hearing aid expert 903 has started to spread. A reason for this is that, for example, prices of hearing aids keep people who need hearing aids away or areas where services of the hearing aid experts 903 can be received are unevenly distributed.

In such a case, for example, as illustrated in FIG. 6, the subject 102 performs the air conduction hearing test via the Internet 119 using an information processing terminal 117. Specifically, in order to perform the air conduction hearing test, for example, dedicated application software may be downloaded and installed in the information processing terminal 117. A hearing aid 116 itself may have a function of the air conduction hearing test.

In such a case, since the hearing aid 116 does not have the bone conduction headset 912, the air conduction hearing test can be performed, but the bone conduction hearing test cannot be performed. For that reason, in a form in which the hearing aid 116 is sold not via the hearing aid expert 903 (a hearing aid sold in such a form is called an OTC (Over-The-Counter) hearing aid or the like), there is a problem in that, although an air conduction hearing level can be measured, a bone conduction hearing level cannot be measured. In other words, in a form in which the hearing aid 116 is sold not via the hearing aid expert 903, there is a problem in that a degree of a conductive hearing loss cannot be measured.

From such a situation, in the form in which the hearing aid 116 is sold not via the hearing aid expert 903, it is assumed that the air conduction hearing level originally indicating both the degree of the conductive hearing loss and the degree of the sensorineural hearing loss indicates the degree of the sensorineural hearing loss, and the hearing aid gain is often set as illustrated on the right side of FIG. 4.

FIG. 7 illustrates an example of a hearing test in a group medical test in a school or a company. The group medical test is mainly aimed at efficiently finding persons having disorder in audibility out of many audibility normal persons, that is, screening. For that reason, in the group medical test, the bone conduction hearing test is not usually performed. In the bone conduction hearing test, it takes a lot of time because a hearing aid is work at a correct wearing position and in a correct wearing state and the test is performed. In addition, in the bone conduction hearing test, it is necessary to perform appropriate masking and the like. Therefore, compared with the air conduction hearing test, a tester who performs the test is required to have a lot of expert knowledge and skills. For that reason, it is difficult to secure an appropriate tester. Therefore, in the group medical test for the main purpose of efficiently finding a person having disorder in audibility among many audibility normal persons, the bone conduction hearing test is usually rarely performed.

However, the present inventors have desired to establish a method of being able to easily grasp a degree of a conductive hearing loss using air conduction sound in order to prevent the subject 901 from missing an opportunity to receive treatment immediately even in the form of selling the hearing aid 116 not via the hearing aid expert 903 or in the group medical test. Further, the present inventors have desired to establish a method of being able to easily grasp a degree of a conductive hearing loss using air conduction sound in order to appropriately set a hearing aid gain even in the form in which the hearing aid 116 is sold not via the hearing aid expert 903. Then, the present inventors have, while repeating earnest studies to obtain such a method, have got an ideal from the following to create the embodiments of the present disclosure.

There is an auditory test that can predict presence or absence of a conductive hearing loss only with air conduction sound. A self-recording audiometer (also called Bekesy-type audiometer) devised in 1947 by Bekesy can provide a material for determining presence or absence of a conductive hearing loss only with the air conduction headset without using the bone conduction headset 912. A characteristic of the self-recording audiometer is that two types of intermittent sound and continuous sound are used as a test sound source. In a test by the self-recording audiometer, the subject 901 continues to press a button while test sound is heard and continues to release the button while the test sound is not heard. Further, the self-recording audiometer gradually reduces the intensity of the test sound while the subject 901 presses the button and gradually increases the intensity of the test sound while the subject 901 releases the button. As a result, when the intensity of the test sound during the test by the self-recording audiometer is recorded, a sawtooth wave graph is observed.

FIG. 8 illustrates an example of an observation result by the self-recording audiometer. When the intermittent sound is used in the self-recording audiometer, the intensity of the recorded test sound transitions across an air conduction hearing threshold of the subject 901. That is, in the self-recording audiometer, a threshold (a median of peaks and troughs of a sawtooth wave) in the case in which the intermittent sound is used as the test sound is substantially equal to a result of the air conduction hearing test by the audiometer of the related art.

Then, the observation result by the self-recording audiometer is classified as follows, and a type of a hearing loss of the subject 901 can be predicted. For example, as illustrated in the upper right side of FIG. 8, when the amplitude (a difference between the peaks and the troughs) of the sawtooth wave observed when the continuous sound is used in the self-recording audiometer decreases compared with the result obtained when the intermittent sound is used, the observation result is classified as the sawtooth wave is classified as Jerger classification type II. In the Jerger classification type II, the replenishment phenomenon is positive and a sensorineural hearing loss due to inner ear disorder is suspected.

On the other hand, when the continuous sound is used, for example, as illustrated in the lower left side of FIG. 8, a phenomenon in which the threshold (the median of the peaks and the troughs of the sawtooth wave) gradually increases with the lapse of time is sometimes observed. In this case, the observation result is classified as a Jerger classification type III. The Jerger classification type III is positive for temporary threshold shift, and sensorineural hearing loss due to a disorder of the posterior labyrinthine is suspected.

For example, as illustrated in the lower center of FIG. 8, when a threshold (a median of peaks and troughs of a sawtooth wave) observed when the continuous sound is used in the self-recording audiometer is lower compared with the result obtained when the intermittent sound is used, the observation result is classified as a Jerger classification type IV. In the Jerger classification type IV, a sensorineural hearing loss due to a disorder of the posterior labyrinthine.

Furthermore, for example, as illustrated in the right of the lower part of FIG. 8, in a case in which a threshold (median value of peaks and troughs of a sawtooth wave) observed when a continuous sound is used in the self-recording audiometer is higher than a result when an intermittent sound is used, the observation result is classified as a Jerger classification type V. In the Jerger classification type V, a psychogenic functional hearing loss is suspected.

Further, for example, as illustrated in the upper left side of FIG. 8, when a decrease in the amplitude of the sawtooth wave does not occur, a transient threshold rise does not occur, and results of the intermittent sound and the continuous sound overlap with each other, the observation result is classified as a Jerger classification type I. The Jerger classification type I is estimated to be normal. Further, although not illustrated in FIG. 8, when a decrease in the amplitude of the sawtooth wave does not occur, the results of the intermittent sound and the continuous sound overlap each other, and a threshold rise occurs, a conductive hearing loss is suspected.

That is, in the self-recording audiometer using the continuous sound and the intermittent sound, an inner-ear hearing loss, a high labyrinthine hearing loss, a sensorineural hearing loss, and a conductive hearing loss can be estimated by performing Jerger classification on the obtained results. Specifically, the inner-ear hearing loss (the sensorineural hearing loss) is estimated in the case of the Jerger classification type II, the retrocochlear hearing loss (the sensorineural hearing loss) is estimated in the case of the Jerger classification type III and the Jerger classification type IV, and the conductive hearing loss is estimated in the case of Jerger classification type I in which the threshold rise occurs.

Subsequently, a correspondence range of the self-recording audiometry with respect to an impaired site is explained. FIG. 9 illustrates a corresponding range of the self-recording audiometry with respect to the impaired site. As illustrated in FIG. 9, a result of the self-recording audiometry by the intermittent sound can be an indicator of a disorder causing a conductive hearing loss, an inner-ear hearing loss, and a retrocochlear hearing loss. However, the conductive hearing loss (a corresponding range 333) and the retrocochlear hearing loss (a corresponding range 335) cannot be distinguished only with the intermittent sound. In addition, when there is an amplitude decrease of the sawtooth wave, the inner-ear hearing loss (a corresponding range 334) can be distinguished because the replenishment phenomenon is positive but, when there is no amplitude decrease of the sawtooth wave, the replenishment phenomenon is not necessarily negative

The result of self-recording audiometry by the continuous sound can also be an indicator of a disorder causing the conductive hearing loss, the inner-ear hearing loss, and the retrocochlear hearing loss. However, the conductive hearing loss (a corresponding range 336) and the retrocochlear hearing loss (a corresponding range 338) cannot be distinguished only with the continuous sound. When there is an amplitude decrease of the sawtooth wave, the replenishment phenomenon is positive and the inner-ear hearing loss (a corresponding range 337) can be distinguished but, when there is no amplitude decrease of the sawtooth wave, the replenishment phenomenon is not necessarily negative. The amplitude decrease of the sawtooth wave is not always the same in the case by the intermittent sound (the corresponding range 334) and the case by the continuous sound (the corresponding range 337).

However, when the threshold in the intermittent sound (the corresponding range 335) and the threshold in the continuous sound (the corresponding range 338) are different, that is, when the transient threshold rise is positive, the retrocochlear hearing loss can be distinguished by comparing the thresholds.

As explained above, the test by the self-recording audiometer (the self-recording audiometry) is considered a test suitable for, when the sensorineural hearing loss is known, classifying the sensorineural hearing loss into the inner-ear hearing loss and the retrocochlear hearing loss. However, in the test by the self-recording audiometer, since different test sounds, that is, the intermittent sound and the continuous sound are used, a degree of the conductive hearing loss cannot be obtained by simple subtraction as in the pure sound audiometry.

As it is seen from the above explanation, the self-recording audiometry is a test method of performing a test using the two types of the test sound, that is, the intermittent sound and the continuous sound and combining the two types of the test sound to specify presence or absence and a place of an auditory disorder. Further, since the self-recording audiometry can be performed only with the air conduction headset, the self-recording audiometry can be carried out by the hearing aid 116. That is, it is likely that the conductive hearing loss can be found by the hearing aid 116.

Thus, the present inventors have created the embodiments of the present disclosure by focusing on a point that a test is performed by giving the two types of the test sound, that is, the intermittent sound and the continuous sound, with the air conduction headset, which is the measurement method by the self-recording audiometry.

However, the Jerger classification performed on the result obtained in the self-recording audiometer using the continuous sound and the intermittent sound also indicates where a disorder is present and does not refer to a degree of the disorder. In particular, in mixed hearing loss, it is difficult to interpret the Jerger classification. That is, the significance of the self-recording audiometry is a minute diagnosis of a sensorineural hearing loss (that is, whether the sensorineural hearing loss is an inner-ear hearing loss or a retrocochlear hearing loss) and is not grasping a degree of a conductive hearing loss. Therefore, a method of capable of easily grasping a degree of the conductive hearing loss using air conduction sound cannot be established only by applying the self-recording audiometry.

Thus, in view of such a situation, the present inventors have created the embodiments of the present disclosure explained below. According to the embodiments of the present disclosure, a degree of the conductive hearing loss can be predicted using an air conduction headset, that is, air conduction sound. As a result, according to the embodiments of the present disclosure, it is possible to recommend the subject 901 to consult a medical institution or the like and it is possible to prevent the subject from missing an opportunity of early treatment for a disorder causing a hearing loss. Further, according to the embodiments of the present disclosure, a gain of the hearing aid 116 of the subject 901 can be appropriately set according to the hearing of the subject 901. Details of such embodiments of the present disclosure are sequentially explained below.

<<2. First Embodiment>>

<2.1 Overview>

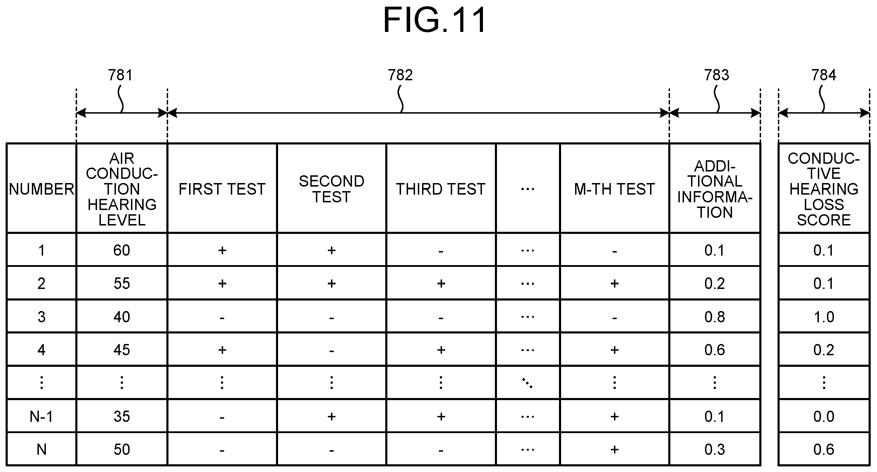

First, an overview of a first embodiment of the present disclosure is explained with reference to FIG. 10 and FIG. 11. FIG. 10 is an explanatory diagram for explaining the overview of the present embodiment and specifically indicates corresponding ranges of impaired sites in auditory tests of first and second groups in the present embodiment. FIG. 11 is an explanatory diagram for explaining learning data according to the present embodiment.

In the present embodiment, auditory tests of two groups are carried out as in the self-recording audiometry using the two types of the test sound, that is, the intermittent sound and the continuous sound.

As illustrated in FIG. 10, an auditory test (a first auditory test) of the first group according to the present embodiment includes an auditory test having a corresponding range 541 and capable of measuring an air conduction audibility threshold. For example, the auditory test of the first group according to the present embodiment can include an air conduction hearing test of a pure sound audiometry and a self-recording audiometry using intermittent sound. Instead of pure sound, warble tone or narrow band noise (specifically referring to noise continuous at a predetermined frequency) may be used. Further, the auditory test of the first group according to the present embodiment may be a combination of a plurality of auditory tests.

As illustrated in FIG. 10, the auditory test of the second group (a second auditory test) according to the present embodiment may have a corresponding range 542, a corresponding range 543, and a corresponding range 544 or may have a corresponding range 545 and a corresponding range 546. Further, the auditory test of the second group according to the present embodiment may have the corresponding range 545 alone or the corresponding range 546 alone.

The auditory test of the second group according to the present embodiment includes an auditory test for the inner ear and/or the posterior labyrinthine. For example, when a self-recording audiometry by intermittent sound is assumed for the auditory test of the first group, the auditory test of the second group can be a self-recording audiometry by continuous sound. In the present embodiment, it is possible to distinguish the retrocochlear hearing loss in the corresponding range 544 in FIG. 10 by comparing a result by the self-recording audiometry by the intermittent sound with a result by the self-recording audiometry by the continuous sound. Further, as explained above, it is possible to distinguish the inner-ear hearing loss in the corresponding range 543 according to presence or absence of observation of a decrease in the amplitude of a sawtooth wave by comparing the result of the self-recording audiometry by the intermittent sound with the result of the self-recording audiometry by the continuous sound. As a result, according to the present embodiment, it is possible to distinguish presence or absence of the conductive hearing loss in the corresponding range 542.

Examples of the auditory test for the inner ear that can be included in the auditory test of the second group according to the present embodiment include an auditory test indicating positive at the time of the inner-ear hearing loss. Specific examples the hearing test include an SISI (Short Increment Sensitivity Index) test, an ABLB (Alternate Binaural Loudness Balance) test, and a DL (Difference Limen) test.

For example, in the SISI test, test sound is output at constant intervals and while intensity is increased by a constant level from time to time to cause the subject 901 to respond when the subject 901 notices that the sound is louder. Then, in the SISI test, the number of correct answers of the subject 901 for the intensity of the sound is scored and an inner-ear hearing loss is estimated based on a score.

For example, in the ABLB test, a pure sound having a frequency to be tested is heard by the left and right ears, a level at which the sound can be heard at the same magnitude on the left and the right is calculated, a lines are connected by results of the left and the right, and an inner-ear hearing loss is estimated according to a gradient of the lines.

Examples of an auditory test of the rear maze that can be included in the auditory test of the second group according to the present embodiment include a TD (Tone Decay) test. For example, the TD test is used to measure auditory fatigue. Continuous sound having audible threshold for a fixed frequency is output to the subject 901 and, when measurement sound becomes inaudible within a predetermined time, a level of the sound is immediately raised by a predetermined amount and output again. This operation is repeated and the retrocochlear hearing loss is estimated based on a level at which the sound is successfully heard for a predetermined time or more.

Further, examples of other auditory tests include a test for estimating a tendency of the retrocochlear hearing loss through comparison with an average pure sound hearing level. Specifically, for example, it is known that a result of a speech recognition threshold test basically coincides with the result of the pure sound audiometry but, in the case of the retrocochlear hearing loss, the result of the speech recognition threshold test more markedly decreases. Therefore, the auditory test of the second group can include the speech recognition threshold test.

In addition, highest speech intelligibility, which is the highest score of a speech discrimination ability test, correlates to an average pure sound hearing level in the case of the sensorineural hearing loss. The highest speech intelligibility tends to decrease when the average pure sound hearing level decreases. In the case of the conductive hearing loss, it is known that the highest speech intelligibility tends to be a score close to 100% even if the average pure sound hearing level decreases. Therefore, the auditory test of the second group can include a highest speech intelligibility test.

A distorted speech hearing test is used for the purpose of detecting a retrocochlear hearing loss because it is known that, when there is a retrocochlear hearing loss, a score of a speech hearing test is lower when there is distortion compared with when there is no distortion. Therefore, the distorted speech hearing test can also be included in the auditory test of the second group.

Further, examples of an auditory test of a posterior labyrinthine that can be included in the auditory test of the second group according to the present embodiment include a binaural separation function test and a sense of direction test. For example, the binaural separation function test is a test that simultaneously presents different auditory stimuli to the left and right ears.

Note that, in the above explanation, several specific names of the tests that can be included in the auditory test of the first group and the auditory test of the second group are exemplified. However, in the present embodiment, the tests that can be included in the auditory test of the first group and the auditory test of the second group are not limited to the specific auditory tests explained above. For example, the tests that can be included in the auditory test of the first group and the auditory test of the second group may be modifications of the specific auditory tests explained above.

Then, in the present embodiment, a degree of the conductive hearing loss is predicted by combining results of the auditory test of the first group and the auditory test of the second group including test contents different from each other. In the present embodiment, the degree of the conductive hearing loss is predicted by using a statistical method for these results or the degree of the conductive hearing loss is predicted by applying a learned model to these results. Accordingly, in the present embodiment, it is possible to easily grasp the degree of the conductive hearing loss using air conduction sound.

Thus, in the present embodiment, in order to predict the degree of conductive hearing loss with the statistical method or to generate the learned model, learning data including known test results is prepared.

FIG. 11 illustrates an example of learning data in the present embodiment. In FIG. 11, N sets of auditory test information 781 of the first group including the test result of the auditory test of the first group, auditory test information 782 of the second group including the test result of the auditory test of the second group, and degree information 784 of the conductive hearing loss at the same period of the same subject 901 are prepared as one set. Note that the auditory test information 781 of the first group illustrated in FIG. 11 includes only one test result. However, in the present embodiment, the test result is not limited to one test result and may be a plurality of test results. Further, the learning data in the present embodiment may include additional information 783. Although only one piece of additional information is illustrated in FIG. 11, the additional information may be a plurality of pieces of additional information.

The auditory test information 781 of the first group can be a test result of an auditory threshold test. More specifically, the auditory test information 781 of the first group can be, for example, an air conduction hearing level. Note that, in the present embodiment, a numerical value of the air conduction hearing level may be used as it is or the numerical value of the air conduction hearing level may be normalized to be between 0.0 and 1.0.

The auditory test information 782 of the second group can be a test result of a test indicating positive in the case of the sensorineural hearing loss. More specifically, the auditory test information 782 of the second group can be, for example, a test result of the self-recording audiometry using the continuous sounds. Note that, in the present embodiment, when a result of the self-recording audiometry using the continuous sound is used as the auditory test information 782 of the second group, it is desirable to perform comparison with a result of the self-recording audiometry using the intermittent sound. For that reason, it is desirable that the auditory test information 781 of the first group include a result of the self-recording audiometry using the intermittent sound.

More specifically, for determination of an auditory disorder of the inner ear, as explained above, the decrease in the amplitude of the sawtooth wave can be used. When the amplitude of the sawtooth wave is 3 dB or less, an auditory disorder of the inner ear is strongly suspected and, when the amplitude is 2 dB or less, it is determined that there is an inner-ear disorder. Therefore, in the present embodiment, for example, a case in which the amplitude is 2.5 dB or less may be defined as positive (+), a case in which the amplitude is more than 2.5 dB may be defined as negative (-), and further, the positive may be defined as 1.0, and the negative may be defined as 0.0. Alternatively, the amplitude may be converted into a score that represents values between positive and negative as continuous values.

As explained above, a transient threshold rise of the continuous sound can be used for the determination of a posterior labyrinthine auditory disorder. Thus, in the present embodiment, for example, when the transient threshold rise of the continuous sound is 10 dB or more with respect to the threshold of the intermittent sound, it may be determined as positive (+) and, when the transient threshold rise is less than 10 dB, it may be determined as negative (-). Further, the positive may be defined as 1.0, and the negative may be defined as 0.0. Alternatively, the amplitude may be converted into a score that represents values between positive and negative as continuous values.

In the present embodiment, when the auditory test of the second group targets both of the inner-ear hearing loss and the retrocochlear hearing loss, an auditory test indicating positive in the case of the inner-ear hearing loss and an auditory test indicating positive in the case of the retrocochlear hearing loss may be combined to form the auditory test of the second group. For example, the auditory test of the second group according to the present embodiment may include a SISI test serving as the auditory test indicating positive in the case of the inner-ear hearing loss and a TD test serving as the auditory test indicating positive in the case of the retrocochlear hearing loss.

Specifically, in the present embodiment, according to the determination criterion of Jerger, for example, a case in which a score of the SISI test is 60% or more may be defined as positive (+), a case in which the score is 20 to 55% may be defined as pseudo-positive (+'), and a case in which the score is 15% or less may be defined as negative (-). Further, positive may be defined as 1.0 and negative may be defined as 0.0 or the score may be converted into a score that represents values between positive and negative as continuous values.

In the present embodiment, in the TD test, for example, a case in which a rising width of a minimum level that can be heard for 1 minute or more is 10 dB or more may be defined as positive (+) and a case in which the raising width is less than 10 dB may be defined as negative (-). Further, positive may be defined as 1.0 and negative may be defined as 0.0 or the score may be converted into a score that represents values between positive and negative as continuous values.

Furthermore, in the present embodiment, the auditory test information 781 of the first group and the auditory test information 782 of the second group may include information obtained by biological sensing for the subject 901. For example, examples of the information of the biological sensing include information of electroencephalograms. More specifically, examples of the information of the electroencephalogram include an auditory evoked response. Specifically, in auditory brainstem response (ABR), which is one of auditory evoked responses, it is clinically known that a reaction classified as an I wave originates in a cochlear nerve (a posterior labyrinthine). Therefore, in the present embodiment, for example, a case in which the height of the I wave of the ABR is less than 0.1 $\mu$V may be defined as positive (+) and a case in which the height is 0.1 $\mu$V or more may be defined as negative (-).

Furthermore, the additional information 783 illustrated in FIG. 11 can be information indicating a statistical tendency because of an auditory state. For example, as the additional information 783, attribute information of the subject 901 such as age, sex, medical history, family history, occupation history, lifestyle, and the like of the subject 901 can be used. For example, as the age is higher, disorders of the inner-ear and the posterior labyrinthine tend to progress with aging. Thus, in the present embodiment, for example, the additional information 783 in which a case in which the age of the subject 901 is 75 years old or more is set as 1.0 and a case in which the age is less than 75 years old is set as 0.0 may be included or information included in the additional information 783 may be a score representing the age by with continuous values of 0.0 to 1.0.

The degree information 784 of the conductive hearing loss is a value representing a degree of the conductive hearing loss. In the present embodiment, for example, an air bone gap can be used as the value representing the degree of the conductive hearing loss. Instead of using the air bone gap as it is, the air bone gap may be converted into a conductive hearing loss score value z and used. In the present embodiment, for example, the conductive hearing loss score value z may be a ratio of the air bone gap to an air conduction hearing level indicated by the following formula (4).

$$z = \text{air bone gap} \diagup \text{air conduction pure sound hearing level} \qquad \cdots (4)$$

The conductive hearing loss score value z is a value of approximately 0.0 to 1.0. Further, when the conductive hearing loss score value z does not fall within a range of 0.0 to 1.0, further arithmetic processing may be performed on the conductive hearing loss score value z to fall within the range of 0.0 to 1.0. In the present embodiment, for example, z = 0.0 may indicate the sensorineural hearing loss, z = 1.0 may indicate the conductive hearing loss, and z more than 0.0 and less than 1.0 may indicate the mixed hearing loss.

Further, in the present embodiment, the degree information 784 of the conductive hearing loss is quantified based on the auditory test information 781 of the first group and the auditory test information 782 of the second group illustrated in FIG. 11. Specifically, for example, an air conduction hearing level included in the auditory test information 781 of the first group is represented as $y_1$, an SISI test score included in the auditory test information 782 of the second group is represented as $y_2$, a TD test score is represented as $y_3$, and an M-th test score included in the auditory test information 782 of the second group is represented as $y_{M+1}$. Then, these values are calculated and set as feature vectors. Further, the conductive hearing loss score value z indicating the degree information 784 of the conductive hearing loss is calculated according to the following formula (5).

$$z = \sum_{i=1}^{M+1} u_i y_i + u_0 \qquad \cdots (5)$$

In the formula (5), $u_i$ is a load coefficient and $u_0$ is a predetermined bias value. Various statistical determination methods can be used to determine the load coefficient $u_i$ and the bias value $u_0$. For example, a large number of known air bone gaps and the auditory test information 781 and 782 of the first and second groups corresponding thereto are acquired and learning data in which the feature vectors and desired score values (for example, 0.0 to 1.0) are paired is prepared based on the air bone gaps and the auditory test information 781 and 782. Further, by performing a multiple regression analysis on the learning data, a linearly approximated load can be obtained and the load coefficient $u_i$ and the bias value $u_0$ can be determined. Note that, in the present embodiment, a neural network may be used instead of the multiple regression analysis or a discrimination method such as a Bayesian estimation method or vector quantization may be used. There is no particular limitation.

Then, in the present embodiment, the conductive hearing loss score value z indicating the degree information 784 of the conductive hearing loss can be calculated from the auditory test information 781 and 782 of the first and second groups by using the formula (5) determined as explained above.

In the above explanation, the conductive hearing loss score value z indicating the degree information 784 of the conductive hearing loss is calculated using the statistical method. However, the present embodiment is not limited to such a method. In the present embodiment, for example, a learned model may be generated using deep machine learning and the conductive hearing loss score value z indicating the degree information 784 of the conductive hearing loss may be calculated using the learned model.

Specifically, for example, a learned model may be generated by performing deep machine learning using a large number of pieces of learning data prepared in advance and the degree information 784 of the conductive hearing loss may be predicted using the generated learned model. Here, one learning sample means an information set in which the auditory test information 781 of the first group, the auditory test information 782 (input data) of the second group, and the degree information 784 (teacher data) of the conductive hearing loss in the same period of the same subject 901 are set as one set.

As explained above, in the present embodiment, by using the statistical method or the method using the learned model explained above, it is possible to predict a degree of a conductive hearing loss using only air conduction sound.

<2.2 Configuration>

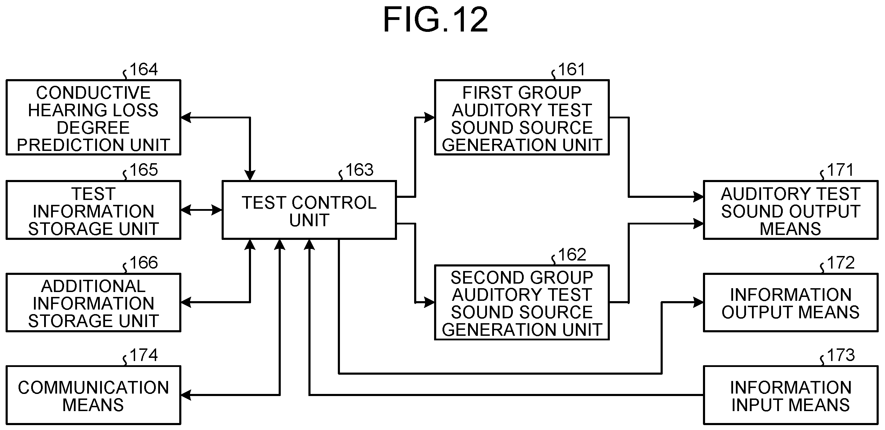

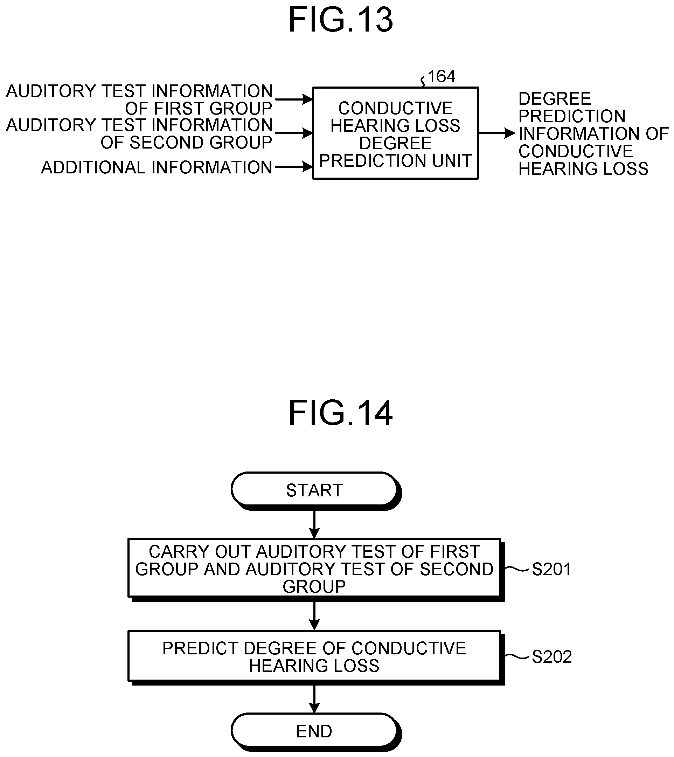

Then, in the present embodiment, the prediction of the degree of conductive hearing loss explained above can be performed using a combination of the information processing terminal 117 and the hearing aid 116 (or the headphone speaker 115). A functional configuration of the information processing terminal 117 according to the present embodiment is explained below with reference to FIG. 12 and FIG. 13. FIG. 12 is a block diagram of the information processing terminal 117 according to the present embodiment and FIG. 13 is an explanatory diagram for explaining a conductive hearing loss prediction unit according to the present embodiment.

As illustrated in FIG. 12, the information processing terminal 117 according to the present embodiment includes a first group auditory test sound source generation unit (a sound source generation unit) 161, a second group auditory test sound source generation unit (a sound source generation unit) 162, a test control unit 163, a conductive hearing loss degree prediction unit (a prediction unit) 164, and a test information storage unit 165. Further, the information processing terminal 117 according to the present embodiment includes an additional information storage unit 166, auditory test sound output means 171, information output means (an output unit) 172, information input means 173, and communication means 174. The functional units included in the information processing terminal 117 according to the present embodiment are explained in order below.

Note that all or a part of the functions of the functional components of the information processing terminal 117 according to the present embodiment explained below are not limited to be executed by the information processing terminal 117 and may be executed by, for example, the hearing aid 116, the headphone speaker 115, or a server on the Internet 119. Specifically, for example, the information processing terminal 117 includes the first group auditory test sound source generation unit 161, the second group auditory test sound source generation unit 162, the test control unit 163, the test information storage unit 165, the additional information storage unit 166, and the communication means 174. The hearing aid 116 or the headphone speaker 115 includes the auditory test sound output means 171. Further, the server on the Internet 119 includes the conductive hearing loss degree prediction unit 164.

(First group auditory test sound source generation unit 161)

The first group auditory test sound source generation unit 161 is controlled by the test control unit 163 explained below and generates a sound source of test sound of the auditory test of the first group. Further, the first group auditory test sound source generation unit 161 outputs the generated sound source to the auditory test sound output means 171. In the present embodiment, when the auditory test of the first group is a combination of a plurality of tests, for example, the first group auditory test sound source generation unit 161 repeats the generation and the output by the number of types of tests.

(Second group auditory test sound source generation unit 162)

The second group auditory test sound source generation unit 162 is controlled by the test control unit 163 and generates a sound source of test sound of the auditory test of the second group. Further, the second group auditory test sound source generation unit 162 outputs the generated sound source to the auditory test sound output means 171. In the present embodiment, when the auditory test of the second group is a combination of a plurality of tests, for example, the second group auditory test sound source generation unit 162 repeats the generation and the output by the number of types of tests.

(Test control unit 163)

The test control unit 163 controls the sound source generation of the first group auditory test sound source generation unit 161 and the second group auditory test sound source generation unit 162 according to input information from the information input means 173 explained below and performs an auditory test according to rules. In order to smoothly advance a test in performing the auditory test, the test control unit 163 may control the information output means 172 explained below and perform guidance such as communicating a procedure or the like to the subject 901.

(Conductive hearing loss degree prediction unit 164)

The conductive hearing loss degree prediction unit 164 can receive auditory tests test results of the first and second groups stored in the test information storage unit 165 explained below through the test control unit 163. The conductive hearing loss degree prediction unit 164 can predict a degree of a conductive hearing loss based on the auditory test results. Further, the conductive hearing loss degree prediction unit 164 may receive the additional information 783 stored in the additional information storage unit 166 explained below via through the test control unit 163 and use the additional information for prediction of a degree of a conductive hearing loss. The conductive hearing loss degree prediction unit 164 can output prediction information of the predicted degree of the conductive hearing loss to the information output means 172 through the test control unit 163.

FIG. 13 illustrates input and output of the conductive hearing loss degree prediction unit 164. The conductive hearing loss degree prediction unit 164 receives input of the auditory test information 781 of the first group and the auditory test information 782 of the second group and outputs conductive hearing loss degree prediction information. Further, the additional information 783 may be input to the conductive hearing loss degree prediction unit 164. Then, the conductive hearing loss degree prediction unit 164 can predict a degree of a conductive hearing loss using only air conduction sound, for example, by using the statistical method or the method using the learned model explained above.

(Test Information storage unit 165)

The test information storage unit 165 stores result information of the auditory test of the first group and result information of the auditory test of the second group according to the control of the test control unit 163.

(Additional information storage unit 166)

The additional information storage unit 166 stores the additional information 783 input via the information input means 173 (specifically, for example, a keyboard, a touch panel, or an external information processing terminal) according to the control of the test control unit 163.

(Auditory test sound output means 171)

The auditory test sound output means 171 can output the sound sources generated by the first group auditory test sound source generation unit 161 and the second group auditory test sound source generation unit 162 toward the subject 901 and execute a test. The auditory test sound output means 171 can be, for example, a receiver of the hearing aid 116.

(Information output means 172)

The information output means 172 can, for example, display the result information of the first group auditory test, the result information of the second group auditory test, information such as the predicted degree of the conductive hearing loss, information derived from these kinds of information, or the like. For example, the information output means 172 can be a screen of the information processing terminal 117 or a display device wirelessly connected to the information processing terminal 117.

(Information input means 173)

For example, a response from the subject 901 is input to the information input means 173. Specifically, for example, when performing the air conduction hearing test, the subject 901 continues to press the response button 914 while hearing sound. At this time, the subject 901 uses the response button 914 as the information input means 173. In the present embodiment, the information input means 173 may be such a response button 914 or may be a touch panel superimposed on a screen of the information processing terminal 117 or other input means.

Further, in the present embodiment, the response is not limited to pressing down the response button 914 and may be a predetermined motion of the head or the arm of the subject 901. In this case, the response may be input by sensing the motion of the subject 901 with an imaging device or an acceleration sensor. In the present embodiment, the response may be voice of the subject 901. In this case, the voice may be collected by a microphone.

Further, in the present embodiment, the response may be input using a biological sensor. For example, a brain wave indicating an auditory brainstem reaction or the like of the subject 901 may be detected by a biological sensor and input as a response. That is, in the present embodiment, the information input means 173 is not particularly limited if the information input means 173 is means that can input information concerning a response or an answer from the subject 901.

(Communication means 174)

The communication means 174 can receive the additional information 783 such as age and sex from external equipment and transmit the additional information to the additional information storage unit 166 through the test control unit 163. Further, the communication means 174 can receive a coefficient, a bias value, a learned model, and the like used in the conductive hearing loss degree prediction unit 164 from the external equipment and transmit the coefficient, the bias value, the learned model, and the like to the conductive hearing loss degree prediction unit 164. In the present embodiment, accordingly, it is possible to always update the coefficient, the bias value, and the learned model to a coefficient, a bias value, and a learned model with higher accuracy. Further, the communication means 174 can transmit prediction information of the degree of the conductive hearing loss predicted by the conductive hearing loss degree prediction unit 164 to the external equipment.

Note that, in the present embodiment, the functional configuration of the information processing terminal 117 is not limited to the configuration illustrated in FIG. 12.

<2.3 Information processing method>

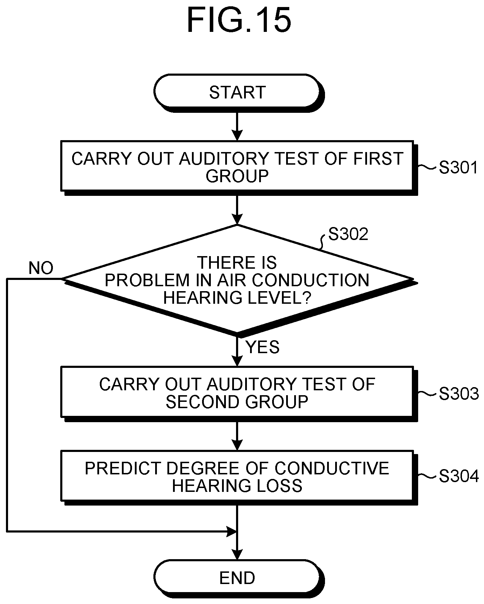

Subsequently, a flow of an information processing method according to the present embodiment is explained with reference to FIG. 14 and FIG. 15. FIG. 14 and FIG. 15 are flowcharts for explaining the flow of the information processing method according to the present embodiment.

In an example illustrated in FIG. 14, a plurality of steps of step S201 and step S202 are included. First, in the example illustrated in FIG. 14, the auditory test of the first group and the auditory test of the second group are carried out (step S201). In the present embodiment, order of performing tests may be determined according to the tests to be performed. For example, since the SISI test is usually performed when an auditory threshold is higher by about 20 dB, it is preferable that an auditory test of the auditory threshold is completed in advance. Therefore, in the present embodiment, the auditory test of the auditory threshold may be carried out first and thereafter the SISI test may be carried out.

Subsequently, a degree of the conductive hearing loss is predicted based on result information of the auditory test of the first group and result information of the auditory test of the second group (step S202) and the processing is ended. As explained above, for example, the formula (4) or the formula (5) can be used to predict the conductive hearing loss.

FIG. 15 illustrates another example. In the example illustrated in FIG. 15, a plurality of steps of step S301 to step S304 are included. The example illustrated in FIG. 15 is different from the example illustrated in FIG. 14 in that, when there is no problem in an air conduction hearing level obtained by the auditory test of the first group, the processing is ended without the auditory test of the second group being carried out. As indicated by the formula (1), the air conduction hearing level is the sum of the degree of the conductive hearing loss and the degree of the sensorineural hearing loss. For this reason, when there is no problem in hearing at the air conduction hearing level, it is seen that there is no problem in the degree of the conductive hearing loss at this point in time. In the example illustrated in FIG. 15, it is possible to reduce a time required for the entire test by ending the auditory test of the second group without performing the hearing test. In particular, in a case such as a group medical test, the reduction of the test time is important.

Specifically, in the example illustrated in FIG. 15, first, the auditory test of the first group is carried out (step S301). Subsequently, it is determined whether there is a problem in an air conduction hearing level from result information of the auditory test of the first group (step S302). As a determination criterion, for example, the determination can be made according to whether the air conduction hearing level is 25 dBHL or more. Then, when there is no problem in the air conduction hearing level (step S302: No), the processing is ended. On the other hand, when there is a problem in the air conduction hearing level (step S302: Yes), the processing proceeds to step S303. Subsequently, the auditory test of the second group is carried out (step S303). Then, a degree of the conductive hearing loss is predicted based on the result information of the auditory test of the first group and result information of the auditory test of the second group (step S304) and the processing is ended. As explained above, for example, the formula (5) can be used to predict the conductive hearing loss.

<2.4 Application example>

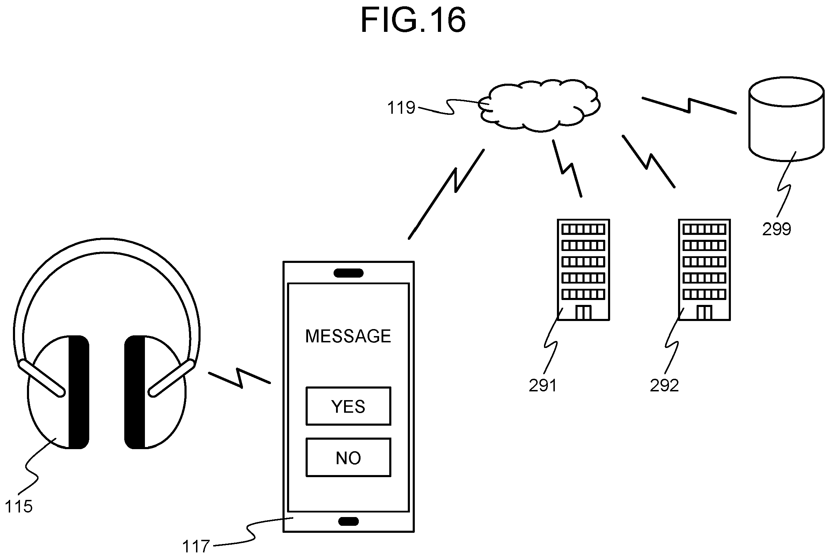

Subsequently, an application example of the present embodiment is explained with reference to FIG. 16. FIG. 16 is an explanatory diagram for explaining an example of an auditory test service in the present embodiment. As explained above, when purchase of the hearing aid 116 is considered, it is preferable to perform an auditory test first in order to grasp a degree of hearing and further determine whether to visit a medical institution or the like before wearing the hearing aid 116.

For example, in the example illustrated in FIG. 16, the subject 901 operates the information processing terminal 117 to access, via the Internet 119, a hearing aid sales site provided by a hearing aid sales company 291. It is assumed that a hearing aid sales site of the hearing aid sales company 291 provides an auditory test service. Note that the auditory test service may be provided by the hearing aid sales company 291 itself or may be provided by an auditory test service providing company 292 different from the hearing aid sales company 291. When the auditory test service providing company 292 provides the auditory test service, a result of the hearing test may be provided from the auditory test service providing company 292 to the hearing aid sales company 291 or may be provided directly from the auditory test service providing company 292 to the subject 901.

Then, the subject 901 operates the information processing terminal 117 to perform the auditory test. In the present application example, a program for performing the auditory test may operate on the Internet 119 or may operate on the information processing terminal 117 and is not particularly limited. In the present application example, the headphone speaker (hereinafter also referred to as headphone) 115 connected to the information processing terminal 117 may not be calibrated for an auditory test. When the headphone 115 not calibrated is used, if the specifications of the headphone 115 do not satisfy a sound pressure level and a frequency characteristic assumed by the auditory test, the auditory test sometimes cannot be accurately carried out. Therefore, in the present application example, it is preferable to acquire characteristic information (output characteristic information) such as a sound pressure level and a frequency characteristic of the headphone 115 in advance before the test. The headphone 115 can be calibrated for the auditory test using such characteristic information. For example, characteristic information of various commercially available headphones 115 is stored in the information processing terminal 117 in advance and the subject 901 selects and inputs information such as a model number and specifications of the headphone 115 owned by the subject 901, whereby the information processing terminal 117 acquires characteristic information corresponding to the headphone 115. Alternatively, the information processing terminal 117 may communicate with the headphone 115, acquire information such as a model number and specifications from the headphone 115, and download the characteristic information of the headphone 115 from a database 299 based on the acquired information. Further, the information processing terminal 117 can perform the auditory test after performing correction based on the acquired characteristic information (specific correction is explained below). Here, the headphone 115 may be either a wired type or a wireless type. The headphone 115 is not limited to an overhead headphone and may be an earphone or a device similar to the earphone.

Note that, in the present application example, the characteristic information of the headphone 115 is not limited to being stored in advance in the information processing terminal 117. The characteristic information of the headphone 115 may be downloaded from the database 299 to the information processing terminal 117 through the Internet 119 or a not-illustrated line. Further, in the present application example, the correction of the headphone 115 may be performed on the information processing terminal 117 or may be performed on a program operating on the Internet 119. Further, the subject 901 can respond or answer to the auditory test using the information processing terminal 117 or the like and receive a test result via the information processing terminal 117 or the like.

Note that, in the example illustrated in FIG. 16, when the characteristic information of the headphone 115 owned by the subject 901 cannot be acquired, the information processing terminal 117 or the like may notify the subject 901 to that effect and recommend use of another headphone 115. Alternatively, when the subject 901 owns a plurality of headphones 115, characteristic information of which is known, the information processing terminal 117 or the like may present the headphone 115 having characteristic information most suitable for the test among the headphones 115 to the subject 901. For example, when the subject 901 owns two headphones, that is, a headphone that can output a strong sound pressure level and a headphone that can output only a weak sound pressure level, the former can cope with a more worsened hearing loss. Therefore, the former is more likely to be suitable for the test. For example, when the subject 901 owns two headphones, that is, an ear covering type overhead headphone and an ear placing type overhead headphone, the former can cope with a noisier environment. Therefore, the former is more likely to be suitable for the test.

In the example illustrated in FIG. 16, a hearing aid purchase desiring person can learn, based on the test result of the auditory test, whether to proceed with purchase consideration of the hearing aid 116 first or to visit a medical institution or the like before proceeding with the purchase consideration. According to the example illustrated in FIG. 16, since such an auditory test service can be used even not via the expert 903, the hearing aid purchase desiring person can determine whether to proceed with the purchase consideration of the hearing aid 116 or to visit a medical institution or the like before proceeding with the purchase consideration. As a result, according to the example illustrated in FIG. 16, it is possible to prevent the subject 901 from missing an opportunity of early treatment of a disorder causing a hearing loss.

Note that, in the explanation with reference to FIG. 16, the application to the auditory test carried out when the purchase of the hearing aid 116 is considered is explained as an example. However, a scene to which the embodiment of the present disclosure can be applied is not limited to the time of the purchase consideration of the hearing aid 116. For example, when desiring to use the auditory test service, the subject 901 can use the auditory test service of the auditory test service providing company 292 using the information processing terminal 117. At this time, the subject 901 can use the headphone 115 owned by the subject 901.

As explained above, in the present embodiment, by combining the results of the auditory test of the first group and the auditory test of the second group including the test contents different from each other and applying the statistical method and the learned model to these results, the degree of the conductive hearing loss can be predicted using only the air conduction sound.

<<3. Second Embodiment>>

<3.1 Overview>

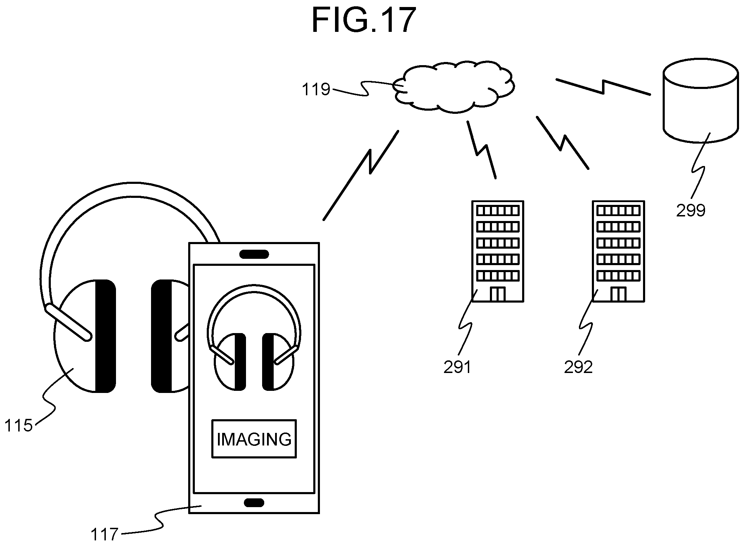

Subsequently, an overview of a second embodiment of the present disclosure is explained with reference to FIG. 17. FIG. 17 is an explanatory diagram for explaining the overview of the present embodiment. In the present embodiment, in the example illustrated in FIG. 16 explained above, the characteristic information of the headphone (the sound output device) 115 to be used can be acquired based on an image of the headphone 115.

Specifically, in the present embodiment, as illustrated in FIG. 17, the headphone 115 owned by the subject 901 is imaged by a camera of the information processing terminal 117 and information such as a model number and specifications of the headphone 115 is specified by an image recognition technology. Then, in the present embodiment, characteristic information of the headphone 115 can be acquired based on the information such as the identified model number and the specifications of the headphone 115 and used for correction at the time of a test. According to the present embodiment, the characteristic information of the headphone 115 can be easily acquired and corrected without bothering the subject 901. Note that the present embodiment can be executed mainly by a program operating on the Internet 119 or a program operating on the information processing terminal 117.

<3.2 Configuration>

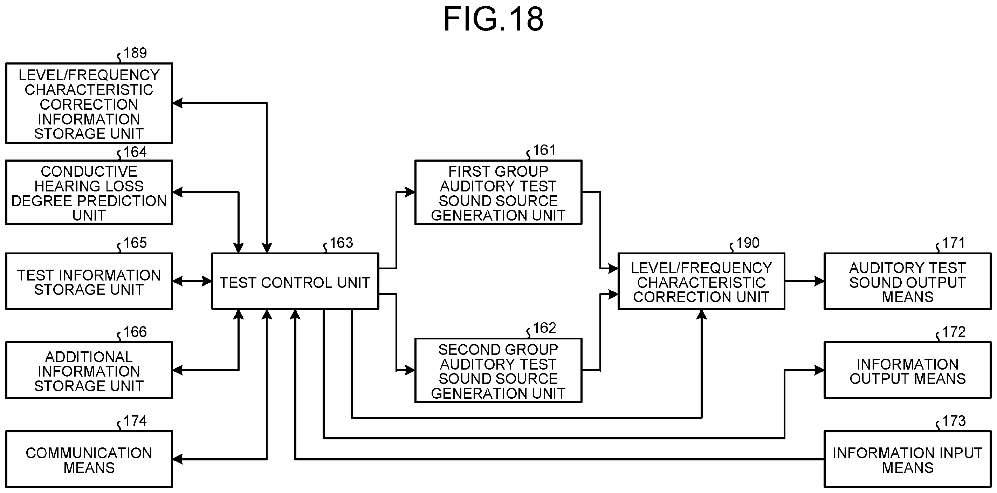

Next, a functional configuration of the information processing terminal 117 according to the present embodiment is explained with reference to FIG. 18. FIG. 18 is a block diagram of the information processing terminal 117 according to the present embodiment.

As illustrated in FIG. 18, as in the first embodiment, the information processing terminal 117 according to the present embodiment includes the first group auditory test sound source generation unit 161, the second group auditory test sound source generation unit 162, the test control unit 163, the conductive hearing loss degree prediction unit 164, and the test information storage unit 165. As in the first embodiment, the information processing terminal 117 according to the present embodiment includes the additional information storage unit 166, the auditory test sound output means 171, the information output means 172, the information input means 173, and the communication means 174. Further, in the present embodiment, the information processing terminal 117 includes a level/frequency characteristic correction information storage unit 189 and a level/frequency characteristic correction unit 190. The functional units included in the information processing terminal 117 according to the present embodiment are explained in order below. Here, explanation is omitted about the functional units common to the first embodiment. Only the level/frequency characteristic correction information storage unit 189 and the level/frequency characteristic correction unit 190 are explained.

Note that all or a part of the functions of the functional components of the information processing terminal 117 according to the present embodiment explained below are not limited to be executed by the information processing terminal 117 and may be executed by, for example, the hearing aid 116, the headphone speaker 115, or a server on the Internet 119. Specifically, for example, the information processing terminal 117 includes the first group auditory test sound source generation unit 161, the second group auditory test sound source generation unit 162, the test control unit 163, the test information storage unit 165, the additional information storage unit 166, the communication means 174, the level/frequency characteristic correction information storage unit 189, and the level/frequency characteristic correction unit 190. The hearing aid 116 or the headphone speaker 115 includes the auditory test sound output means 171. Further, the server on the Internet 119 includes the conductive hearing loss degree prediction unit 164.

(Level/frequency characteristic correction information storage unit 189)

The level/frequency characteristic correction information storage unit 189 stores images of various headphones 115 in linkage with model numbers of the headphones 115. The images are used when the level/frequency characteristic correction unit 190 specifies a model number of the headphone 115 using an image recognition technology. The level/frequency characteristic correction information storage unit 189 also stores characteristic information of the various headphones 115. The characteristic information is used when the level/frequency characteristic correction unit 190 performs correction. Note that the information stored in the level/frequency characteristic correction information storage unit 189 may be downloaded from the database 299 through the Internet 119 or a not-illustrated line. Note that, In the present embodiment, the level/frequency characteristic correction information storage unit 189 is not limited to storing the images of the headphones 115 and may store identification signals and the like output from the headphones 115. In this case, by collating an identification signal and the like stored in the level/frequency characteristic correction information storage unit 189 and an identification signal and the like acquired from the headphone 115, information such as a model number and specifications of the headphone 115 can be specified.

(Level/frequency characteristic correction unit 190)

The level/frequency characteristic correction unit 190 can correct, based on the characteristic information of the headphone 115 stored in the level/frequency characteristic correction information storage unit 189, the sound source of the test sound generated by the first group auditory test sound source generation unit 161 and the second group auditory test sound source generation unit 162. Further, the level/frequency characteristic correction unit 190 can output the corrected test sound source to the auditory test sound output means 171.

When the characteristic information of the headphone 115 is acquired, the level/frequency characteristic correction unit 190 may specify a model number of the headphone 115 based on an image of the headphones 115 using an image recognition technology.

Note that, in the present embodiment, the functional configuration of the information processing terminal 117 is not limited to the configuration illustrated in FIG. 18.

<3.3 Information processing method>

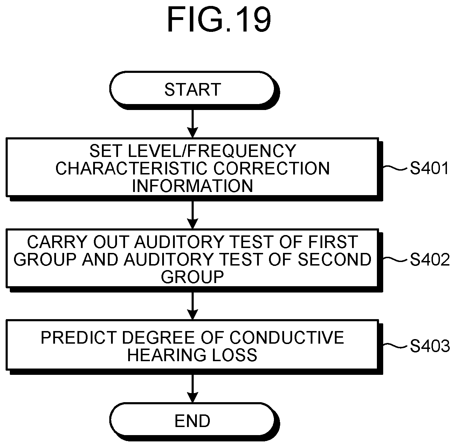

Subsequently, a flow of an information processing method according to the present embodiment is explained with reference to FIG. 19. FIG. 19 is a flowchart illustrating a flow of the information processing method according to the present embodiment.

As illustrated in FIG. 19, the information processing method according to the present embodiment includes a plurality of steps of step S401 to step S403. First, level/frequency characteristic correction information is set (step S401). Specifically, a model number of the headphone 115 is specified by applying the image recognition technology to an image of the headphone 115 owned by the subject 901 captured by the camera of the information processing terminal 117. Then, characteristic information of the headphone 115 is acquired based on the specified model number of the headphone 115. Level/frequency characteristic correction information for correcting the test sound source is set based on the acquired information. Further, the sound source of the test sound is corrected based on the set level/frequency characteristic correction information. Note that, in the present embodiment, when characteristic information of the headphone 115 used by the subject 901 cannot be acquired, the subject 901 is notified to that effect and the processing is ended.

Further, in the present embodiment, steps S402 and S403 are carried out in order. However, these steps are the same as steps S201 and S202 of the information processing method according to the first embodiment illustrated in FIG. 14. Therefore, explanation of the steps is omitted here.

As explained above, in the present embodiment, since the headphone 115 owned by the subject 901 is imaged by the camera of the information processing terminal 117 and the model number of the headphone 115 is specified by the image recognition technology, the characteristic information of the headphone 115 can be easily acquired and corrected without bothering the subject 901.

<<4. Third Embodiment>>

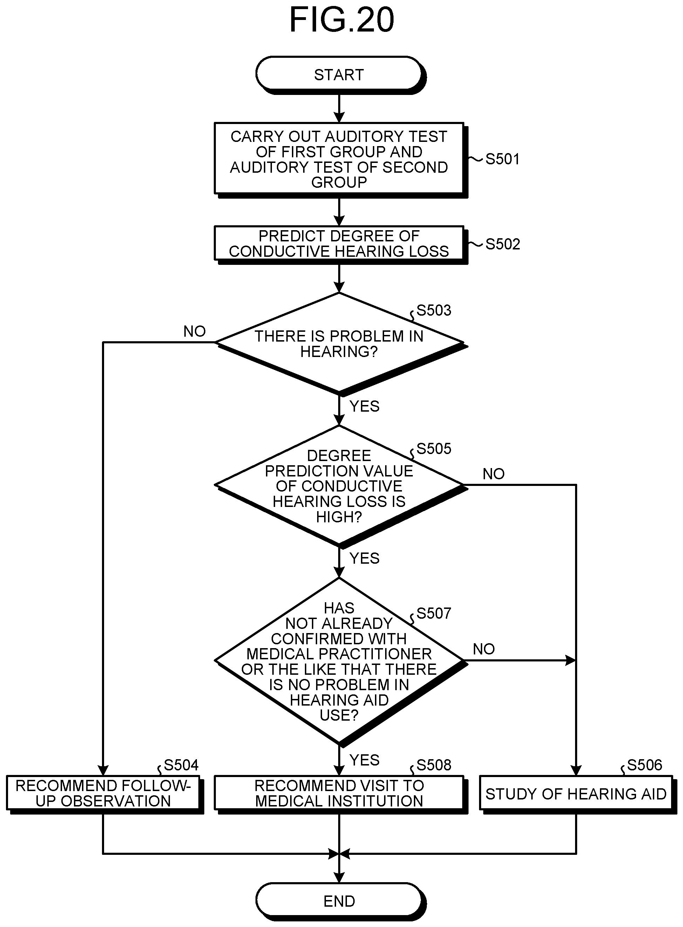

Subsequently, an overview of a third embodiment of the present disclosure is explained with reference to FIG. 20. FIG. 20 is a flowchart for explaining a flow of an information processing method according to the present embodiment. The present embodiment is an application example at the time when necessity of a medical institution visit before hearing aid wearing is determined. In the present embodiment, a case in which the subject 901 performs a test by himself/herself is mainly assumed.

As illustrated in FIG. 20, the information processing method according to the present embodiment includes a plurality of steps of step S501 to step S508.

First, as in the embodiments explained above, the auditory test of the first group and the auditory test of the second group are carried out (step S501). Subsequently, a degree of a conductive hearing loss is predicted based on result information of the auditory test of the first group and the result information of the auditory test of the second group (step S502).

Then, it is determined whether there is a problem in hearing ability (step S503). In step S503, it is determined whether a level is a level for which hearing aid wearing is appropriate. In the present embodiment, for example, whether an air conduction hearing level is 40 dBHL or more can be used as a criterion. When it is determined that there is no problem in hearing (step S503: No), the processing proceeds to step S504. Then, it is recommended to the subject 901 to periodically perform follow-up observation according to necessity (step S504) and the processing is ended.

On the other hand, when it is determined that there is a problem in hearing (step S503: Yes), the processing proceeds to step S505. Then, it is determined whether a degree prediction value of a conductive hearing loss is high (step S505). In the present embodiment, for example, when the degree prediction value of the conductive hearing loss is 25 dBHL or more, a criterion for determining that the degree prediction value of the conductive hearing loss is high can be used. In such a case, the predicted degree of the conductive hearing loss is compared with a reference value (a predetermined threshold) of 25 dBHL to make determination. When it is determined that the degree prediction value of the conductive hearing loss is not high (step S505: No), the processing proceeds to step S506. Then, the subject 901 is recommended to consider wearing a hearing aid (step S506) and the processing is ended.

When it is determined that the degree prediction value of the conductive hearing loss is high (step S505: Yes), the processing proceeds to step S507. Then, the subject 901 is asked whether the subject 901 has already confirmed with a medical practitioner or the like that there is no problem in hearing aid wearing (step S507). For example, when the subject 901 has finished treatment of the conductive hearing loss and still has the conductive hearing loss, it is highly likely that it is determined that there is no problem in hearing aid wearing. In the case of an answer that the confirmation with the medical practitioner or the like has been finished (step S507: No), the processing proceeds to step S506. On the other hand, in the case of an answer that the confirmation with the medical practitioner or the like has not been finished (step S507: Yes), the processing proceeds to step S508. Further, the subject 901 is recommended to visit a medical institution or the like (step S508) and the processing is ended.

Note that, in the flowchart illustrated in FIG. 20, whether there is a problem in hearing is determined in step S503. However, the present embodiment is not limited to this. In the present embodiment, for example, as illustrated in FIG. 15, whether there is a problem in hearing may be determined between the auditory test of the first group and the auditory test of the second group.

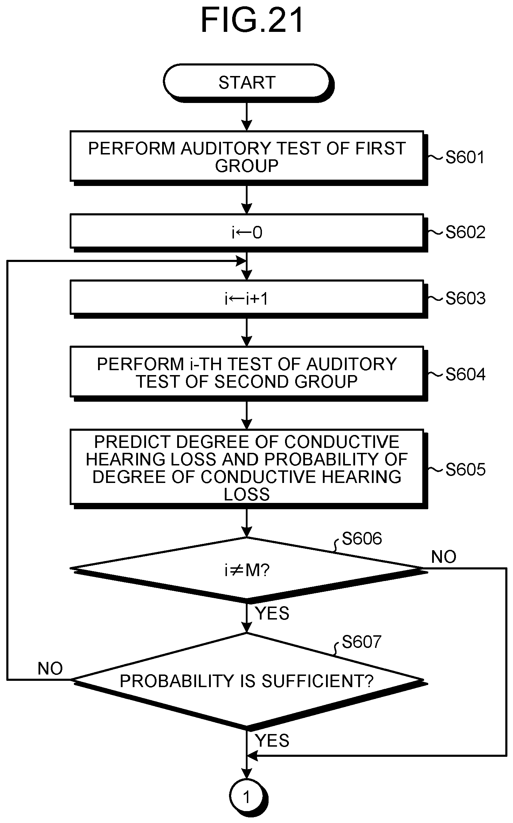

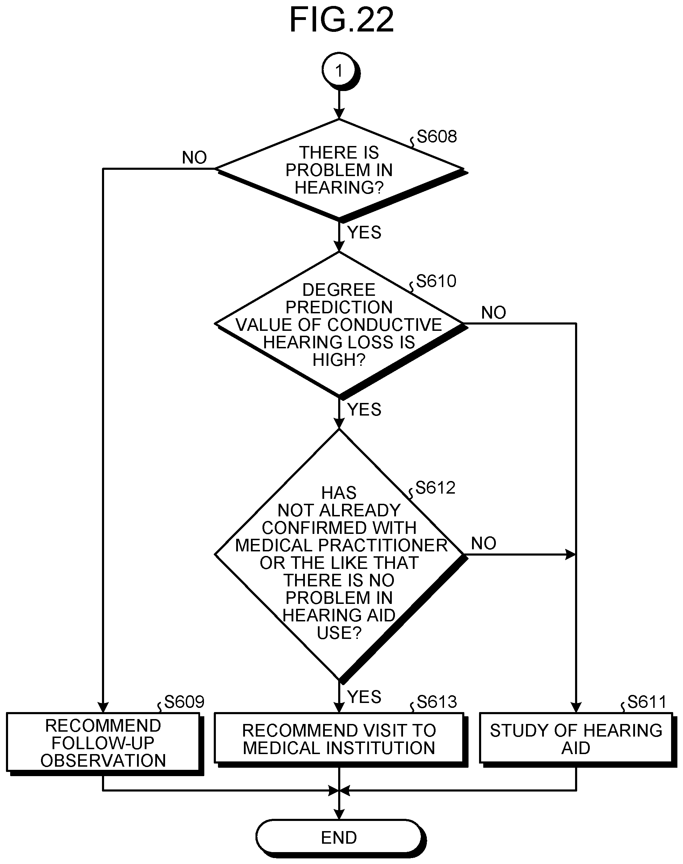

The information processing method according to the present embodiment can be modified. A modification is explained with reference to FIG. 21 and FIG. 22. FIG. 21 and FIG. 22 are flowcharts for explaining a flow of an information processing method according to the modification of the present embodiment. As illustrated in FIG. 21 and FIG. 22, the information processing method according to the present modification includes a plurality of steps of step S601 to step S613.

Specifically, in the present modification, first, the auditory test of the first group is carried out (step S601). Subsequently, 0 is input to a count i of the auditory test of the second group (step S602). Further, 1 is added to the count i of the auditory test of the second group (step S603).

Subsequently, an i-th auditory test in the auditory test of the second group including a plurality of auditory tests is performed (step S604). Then, a degree of the conductive hearing loss is predicted based on result information of the auditory test of the first group and result information of the auditory test of the second group already performed and probability of prediction of the degree of conductive hearing loss is predicted (step S606). In the present modification, for example, the likelihood may be estimated as probability of prediction of the degree of the conductive hearing loss. In this case, by performing the deep machine learning using a large number of known learning data used for generating the learned model explained above, an estimation model for estimating likelihood may be generated and likelihood of a degree of the conductive hearing loss predicted anew may be estimated using the estimation model.

Subsequently, it is determined whether the count i of the auditory test of the second group is a predetermined value M (step S606). The predetermined value M may be, for example, the same as the number of auditory tests included in the auditory test of the second group or may be set by a user as appropriate. When the count i of the hearing tests of the second group is not the predetermined value M (step S606: Yes), the processing proceeds to step S607. When the count i of the auditory test of the second group is the predetermined value M (step S606: No), the processing proceeds to step S608 illustrated in FIG. 22.

Subsequently, it is determined whether the probability of the prediction of the degree of the conductive hearing loss predicted in step S605 is sufficient probability (step S607). For example, the probability of the prediction of the degree of the conductive hearing loss can be determined by being compared with a predetermined threshold. When the probability of the prediction of the predicted degree of the conductive hearing loss is sufficient (step S607: Yes), the processing proceeds to step S608 illustrated in FIG. 22. On the other hand, when the probability of the prediction of the predicted degree of the conductive hearing loss is not sufficient (step S607: No), the processing returns to step S603. From step S603, the next (i+1-th) auditory test in the auditory test of the second group including the plurality of auditory tests is performed and a degree of the conductive hearing loss is predicted using result information of the hearing tests of the second group larger in number than the last time. Therefore, possibility that the probability of the prediction of the degree of the conductive hearing loss is improved increases.

Further, step S608 to step S613 illustrated in FIG. 22 are carried out. However, these steps are the same as step S502 to step S508 of the information processing method according to the present embodiment explained with reference to FIG. 20. Therefore, explanation of the steps is omitted here.

In the present modification, the number of tests of the auditory test of the second group is increased until the prediction of the degree of the conductive hearing loss has constant probability. Therefore, according to the present modification, since the auditory test of the second group is ended at a stage when the prediction of the degree of the conductive hearing loss having the constant probability is obtained, that is, all the auditory tests of the second group are not always performed every time, a time required for the entire test can be reduced.

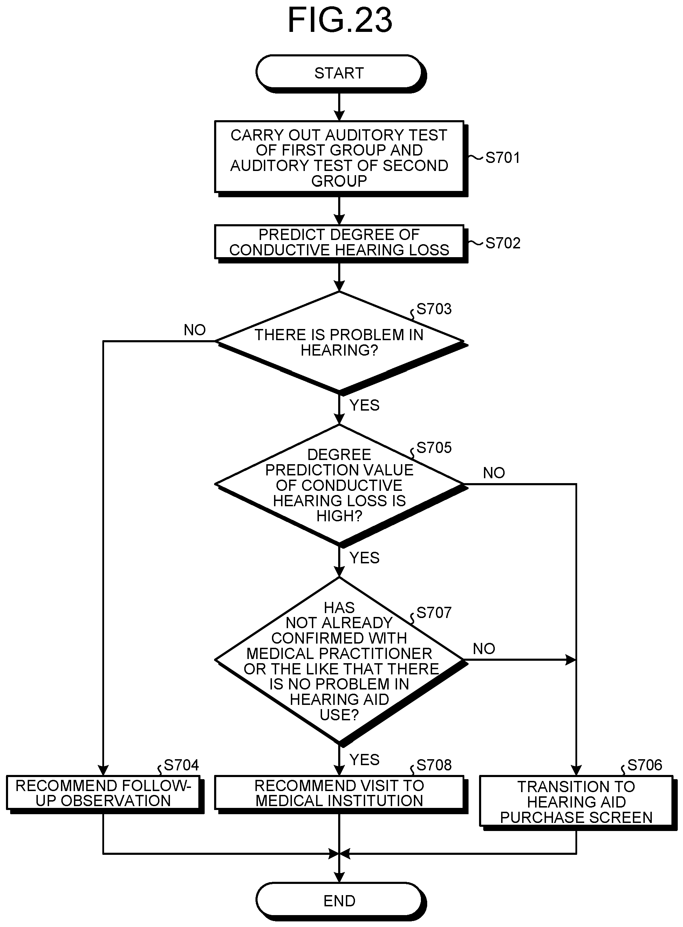

Subsequently, with reference to FIG. 23, an embodiment is explained in which, when it has already been confirmed with a medical practitioner or the like that the degree prediction value of the conductive hearing loss is not high or there is no problem in hearing aid wearing even if the degree prediction value of the conductive hearing loss is high, a screen automatically transitions to a hearing aid purchase screen. FIG. 23 is a flowchart for explaining a flow of the information processing method according to the present embodiment. Note that, in the present embodiment, a purchase button may become usable instead of the screen transitioning to the hearing aid purchase screen. In the present embodiment, since the subject 901 is always subjected to the auditory test before the screen transitions to the hearing aid purchase screen, it is possible to prevent the subject 901 from purchasing the hearing aid 116 without noticing the auditory test service or carelessly purchasing the hearing aid 116 regardless of the fact that the subject 901 is recommended to visit a medical institution or the like in the auditory test service.

As illustrated in FIG. 23, the information processing method according to the present embodiment includes a plurality of steps of step S701 to step S708. Note that steps other than step S706 are the same as step S501 to step S505 and step S507 and step S508 of the information processing method according to the present embodiment explained with reference to FIG. 20. Therefore, explanation of the steps is omitted here.

In the present embodiment, the screen transitions to the hearing aid purchase screen (step S706) and the process is ended. On the hearing aid purchase screen, a purchase procedure can be performed.

Note that, in the flowchart illustrated in FIG. 23, whether there is a problem in hearing is determined in step S703. However, the present embodiment is not limited to this. In the present embodiment, for example, as illustrated in FIG. 15, whether there is a problem in hearing may be determined between the auditory test of the first group and the auditory test of the second group.

Incidentally, when an article or service is purchased on a website, a procedure is sometimes interrupted in the middle. Based on such a case, for example, although the auditory test is performed according to the flow of FIG. 23, the processing reaches step S706, and the screen transitions to the hearing aid purchase screen, access to the website is sometimes interrupted for some reason. Thereafter, if the subject 901 has to be subjected to the auditory test again when the subject 901 attempts to resume the purchase procedure, it is likely that the willingness of the subject 901 to purchase the hearing aid 116 is greatly reduced. Thus, in the present embodiment, for example, this is avoided by a configuration in which the processing can be immediately skipped to step S706 according to necessity. For example, when the processing reaches step S706 once, information concerning the reaching step S706 is stored and the information is confirmed at the time of next access to resume the processing from step S706. Note that, in such a case, since hearing changes with the lapse of time, an elapsed time (specifically, an elapsed period starting from the last test) for permitting the processing to be immediately resumed from step S706 may be determined.

As explained above, in the present embodiment, since the subject 901 can perform the test by himself/herself and predict the degree of the conductive hearing loss, even in a place such as the home of the subject 901, the subject 901 can learn whether to proceed with purchase consideration of the hearing aid 116 or to visit a medical institution or the like before the purchase consideration. As a result, according to the present embodiment, it is possible to prevent the subject 901 from missing an opportunity of early treatment of a disorder causing hearing loss. Further, according to the present embodiment, even when the hearing aid 116 is purchased through the Internet 119, since a degree of a conductive hearing loss of a purchase desiring person can be predicted, it is possible to prevent a purchase desiring person for whom hearing aid wearing is inappropriate from purchasing the hearing aid 116.

<<5. Fourth Embodiment>>

<5.1 Configuration>

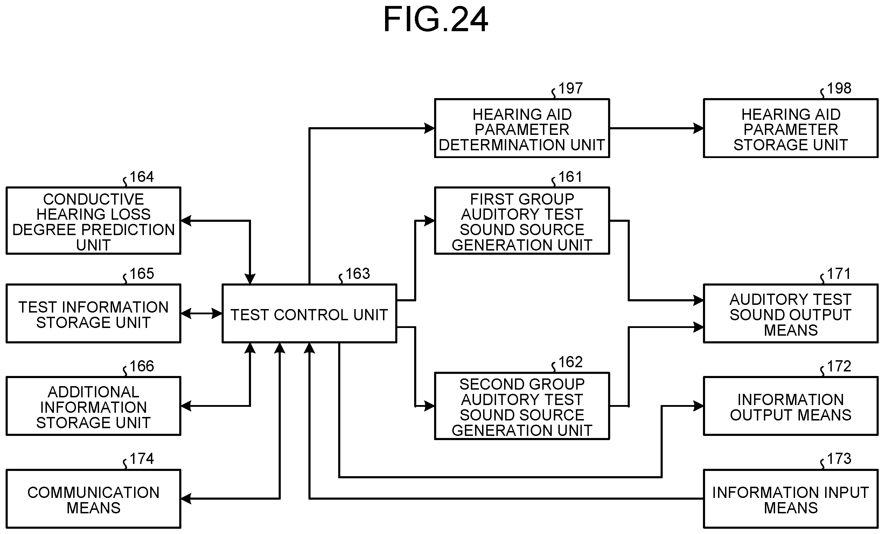

Subsequently, an overview of a fourth embodiment of the present disclosure is explained. The present embodiment is an embodiment in which a hearing aid parameter for the hearing aid 116 can be set. First, a functional configuration of the information processing terminal 117 according to the present embodiment will be described with reference to FIG. 24. FIG. 24 is a block diagram of the information processing terminal 117 according to the present embodiment.

As illustrated in FIG. 24, as in the first embodiment, the information processing terminal 117 according to the present embodiment includes the first group auditory test sound source generation unit 161, the second group auditory test sound source generation unit 162, the test control unit 163, the conductive hearing loss degree prediction unit 164, and the test information storage unit 165. As in the first embodiment, the information processing terminal 117 according to the present embodiment includes the additional information storage unit 166, the auditory test sound output means 171, the information output means 172, the information input means 173, and the communication means 174. Further, in the present embodiment, the information processing terminal 117 includes a hearing aid parameter determination unit 197 and a hearing aid parameter storage unit 198. The functional units included in the information processing terminal 117 according to the present embodiment are explained in order. However, here, explanation is omitted about the functional units common to the first embodiment and only the hearing aid parameter determination unit 197 and the hearing aid parameter storage unit 198 are explained.

Note that all or a part of the functions of the functional components of the information processing terminal 117 according to the present embodiment explained below are not limited to be executed by the information processing terminal 117 and may be executed by, for example, the hearing aid 116, the headphone speaker 115, or a server on the Internet 119. Specifically, for example, the information processing terminal 117 includes the first group auditory test sound source generation unit 161, the second group auditory test sound source generation unit 162, the test control unit 163, the test information storage unit 165, the additional information storage unit 166, and the communication means 174. The hearing aid 116 or the headphone speaker 115 includes the auditory test sound output means 171 and the hearing aid parameter storage unit 198. Further, the server on the Internet 119 includes the conductive hearing loss degree prediction unit 164 and the hearing aid parameter determination unit 197.

(Hearing aid parameter determination unit 197)

The hearing aid parameter determination unit 197 can acquire, through the test control unit 163, degree prediction information of a conductive hearing loss predicted by the conductive hearing loss degree prediction unit 164 and determine a hearing aid parameter, which is a setting parameter of the hearing aid 116, based on the predicted degree of the conductive hearing loss. The hearing aid parameter can be a parameter used for gain setting control and noise suppression setting control (noise suppression strength, a frequency characteristic, and the like) of the hearing aid 116. More specifically, the hearing aid parameter may be a parameter of gain setting, such as the fourth section 754 illustrated in FIG. 4. For example, a generally widely used scheme NAL-NL2 (National Acoustic Laboratories Nonlinear 2), DSLv5 (Desired Sensation Level version 5), or the like can be used to determine the hearing aid parameter. These schemes use an air conduction hearing level and a bone conduction hearing level as input data. If there are an air conduction hearing level (result information of the auditory test of the first group) and prediction information of a degree of the conductive hearing loss, the bone conduction hearing level can be calculated from the formula (1) and the formula (2) described above.

In addition, for example, a person having a sensorineural hearing loss tends to have more difficulty in listening under noise than a person having conductive hearing loss. Therefore, the hearing aid parameter determination unit 197 may determine intensity of noise suppression and a frequency characteristic using degree prediction information of a conductive hearing loss and degree prediction information of a sensorineural hearing loss. More specifically, for example, the hearing aid parameter determination unit 197 increases the intensity of the noise suppression when a degree of the sensorineural hearing loss is large. Further, the hearing aid parameter determination unit 197 outputs the determined hearing aid parameter to the hearing aid parameter storage unit 198.

(Hearing aid parameter storage unit 198)

The hearing aid parameter storage unit 198 stores the hearing aid parameter determined by the hearing aid parameter determination unit 197.

Note that, in the present embodiment, the functional configuration of the information processing terminal 117 is not limited to the configuration illustrated in FIG. 24.

<5.2 Information processing method>

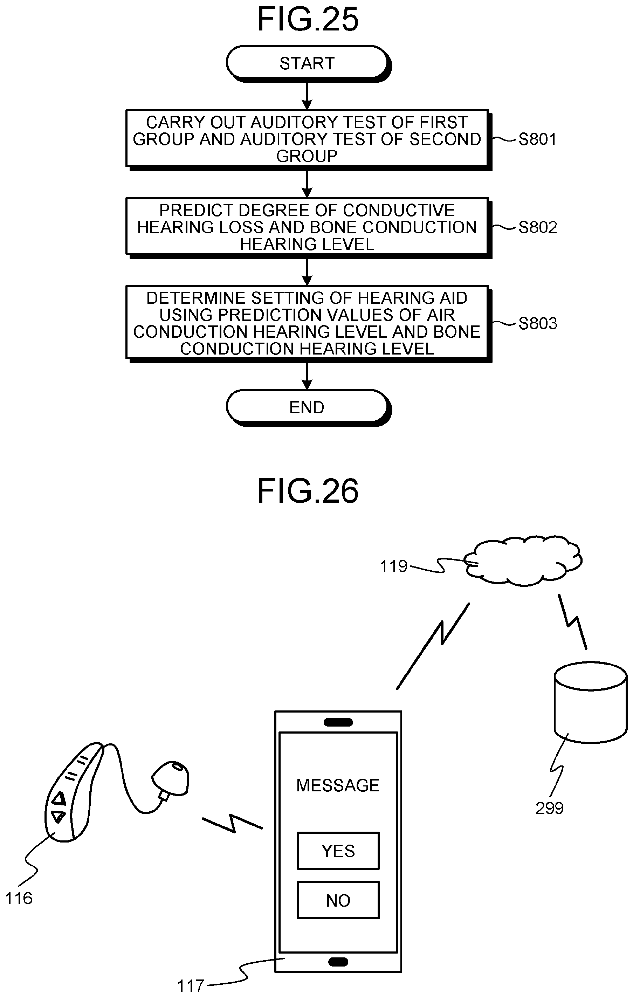

Subsequently, a flow of an information processing method according to the present embodiment is explained with reference to FIG. 25 and FIG. 26. FIG. 25 is a flowchart for explaining the flow of the information processing method according to the present embodiment. FIG. 26 is an explanatory diagram for explaining the present embodiment. As illustrated in FIG. 25, the information processing method according to the present embodiment includes a plurality of steps of step S801 to step S803.

First, as in the embodiments explained above, the auditory test of the first group and the auditory test of the second group are carried out (step S801). Subsequently, a degree of a conductive hearing loss is predicted based on result information of the auditory test of the first group and result information of the auditory test of the second group. At this time, in the present embodiment, a bone conduction hearing level is also predicted (step S802). If there are prediction values of an air conduction hearing level (auditory test information of the first group) and a degree of a conductive hearing loss, a prediction value of a bone conduction hearing level can also be calculated from the formula (1) and the formula (2) described above.

Then, a hearing aid parameter, which is setting of the hearing aid 116, is determined based on the prediction values of the air conduction hearing level and the bone conduction hearing level (step S803). For example, when gain setting of the hearing aid 116 is determined, it is possible to use a generally widely used scheme NAL-NL2 or DSLv5. Further, in the present embodiment, the hearing aid parameter may be determined using a unique scheme.

Then, as illustrated in FIG. 26, the information processing terminal 117 performs a hearing test using the hearing aid 116 and obtains degree prediction information of the conductive hearing loss. Then, the information processing terminal 117 registers the result information of the auditory test, the additional information 783, and the degree prediction information of the conductive hearing loss in the database 299 on the Internet 119. That is, the database 299 on the Internet 119 stores various kinds of information. Then, the information stored in the database 299 can be used to improve accuracy of degree prediction of the conductive hearing loss. For example, by comparing a relation between an average and a variance of respective kinds of auditory test information in learning data used for creating a learned model and respective kinds of auditory test information with the relation in the information stored in the database 299, a difference from the learning data can be known, leading to improvement of the learning data.

Further, the information transmitted from the information processing terminal 117 to the database 299 may include log data concerning fine adjustment of the hearing aid 116. The difference from the log data in the case of the degree prediction value of the conductive hearing loss can be grasped by comparing with the separately prepared fine-adjustment log data in the case of performing the bone conduction hearing test, and the learning data can be improved.

<5.3 Display example>

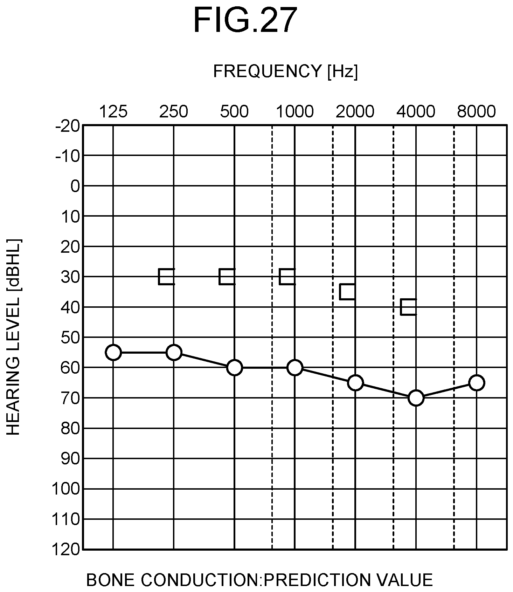

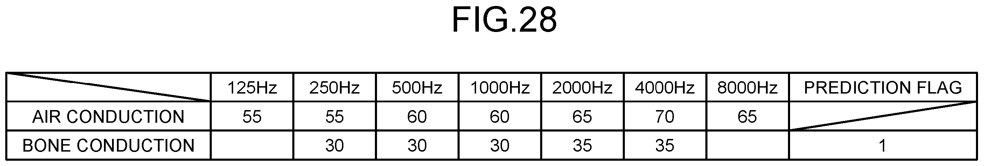

Subsequently, a display example in the present embodiment is explained with reference to FIG. 27 and FIG. 28. FIG. 27 and FIG. 28 are explanatory diagrams for explaining a display example according to the present embodiment.

FIG. 27 is a display example at the time when a prediction value of a bone conduction hearing level is displayed on an audiogram. The prediction value of the bone conduction hearing level can be calculated using the formula (1) and the formula (2) described above using the air conduction hearing level (the result information of the auditory test of the first group) and the degree prediction value of the conductive hearing loss. The audiogram is a display method most widely used when displaying hearing. In the present embodiment, the hearing can be displayed in the form of the audiogram using the prediction value of the bone conduction hearing level.

In the present embodiment, it is preferable that the audiogram is displayed such that it is seen that the bone conduction hearing level is the prediction value. For example, in the example illustrated in FIG. 27, it is indicated together, outside a field of the audiogram, that the bone conduction hearing level is the prediction value. Accordingly, a person who looks at the audiogram can recognize that the bone conduction hearing level described in the audiogram is the prediction value. Note that, in the present embodiment, a display form is not limited to the form illustrated in FIG. 27.

FIG. 28 is a display example at the time when the prediction value of the bone conduction hearing level is represented as data. In the present embodiment, the prediction value may be represented as data inside equipment that has predicted the prediction value, may be represented as data at the time of data communication between different kinds of equipment, or may be represented as data in different kinds of equipment and is not particularly limited. In the example illustrated in FIG. 28, a "prediction flag" indicating that the bone conduction hearing level is the prediction value is included. Specifically, for example, it is decided that 1 indicates the prediction value and 0 indicates a measurement value. In this way, even in a device different from the device in which the bone conduction hearing level is predicted, it is possible to distinguish whether it is a prediction value or a measurement value. In FIG. 28, the prediction flag is added to the bone conduction hearing level. However, in the present embodiment, prediction flags may be added to an air bone gap, a degree of the conductive hearing loss, and a degree of the sensorineural hearing loss such that it can be seen that the air conduction difference, the degree of the conductive hearing loss, and the degree of the sensorineural hearing loss are prediction values. Accordingly, for example, equipment that has received data added with a prediction flag can change content of processing between measurement and prediction.

As explained above, in the present embodiment, since the subject 901 can perform the test by himself or herself and predict the degree of the conductive hearing loss, the subject 901 can suitably set the hearing aid 116 in a place like the home of the subject 901.

<5.4 Application example>

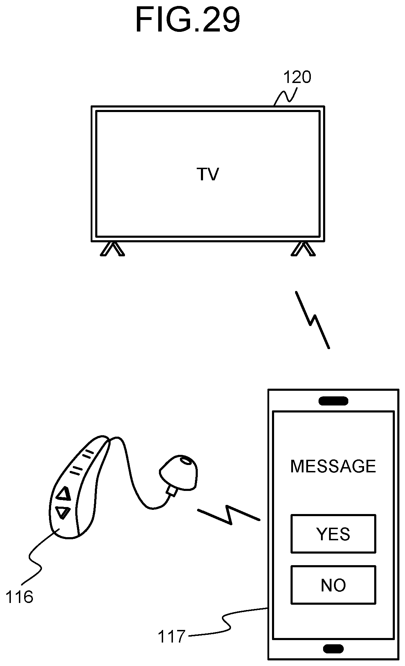

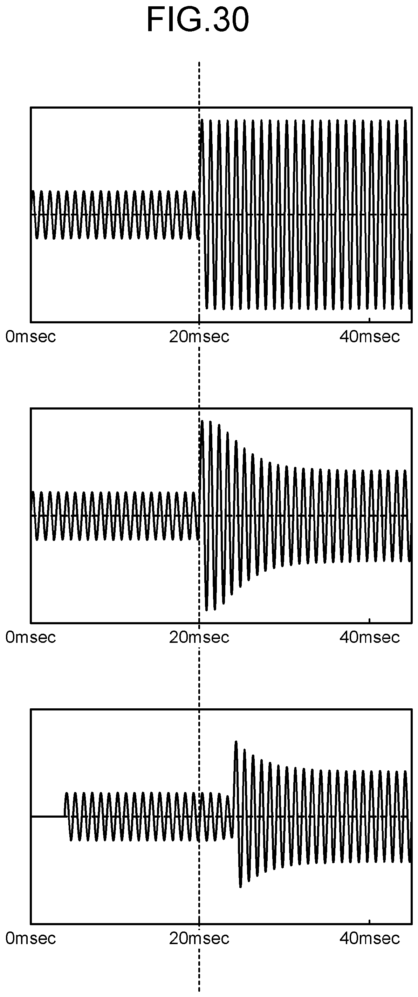

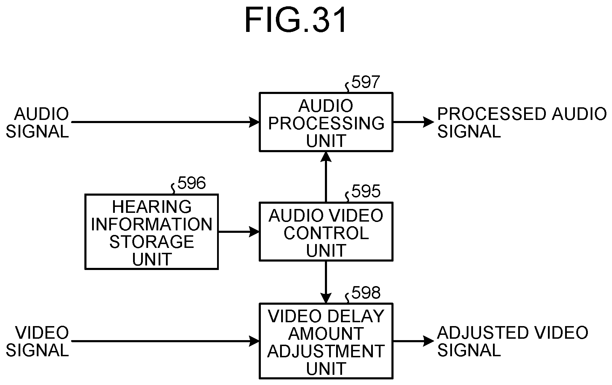

An application example in which hearing information is used in external equipment other than the hearing aid 116 is explained with reference to FIG. 29 to FIG. 31. FIG. 29 and FIG. 30 are explanatory diagrams for explaining an application example according to the present embodiment. FIG. 31 is a block diagram of an external device according to the present embodiment.

In the present application example, as illustrated in FIG. 29, the information processing terminal 117 acquires degree prediction information of a conductive hearing loss and an air conduction hearing level of the subject 901 and thereafter transmits these kinds of information to an external equipment 120. Alternatively, the information processing terminal 117 may acquire degree prediction information of a conductive hearing loss and an air conduction hearing level of the subject 901 and thereafter determine a parameter for controlling the external equipment 120 and transmit the determined parameter to the external equipment 120. In the example illustrated in FIG. 29, the external equipment 120 is a television device (an acoustic device). Note that, in the present embodiment, the external equipment 120 is not limited to the television device (the acoustic device). The external equipment 120 can receive information from the information processing terminal 117 and change content of processing in the external equipment 120. In the following explanation, two examples of processing performed by the external equipment 120 are explained.

As a first example, the external equipment 120 can weaken, for example, for the subject 901 having a strong degree of the sensorineural hearing loss, a signal level channels other than an audio channel compared with the audio channel in a multichannel signal of video content. The channel may be an object instead. In the case of the sensorineural hearing loss, frequency discrimination ability tends to decrease. Therefore, when sound other than voice overlaps with the voice, the subject 901 often felts difficulty in listening to the voice stronger than a normal listener. Although some of the hearing aids 116 have a function of sound enhancement processing, it is possible to more reliably improve listening by not outputting, from the television device, sound that is likely to disturb listening. Therefore, in the present application example, the external equipment 120 performs, based on the hearing information such as the degree prediction information of the conductive hearing loss of the subject 901, the air conduction hearing level, and the parameter transmitted from the information processing terminal 117 or the like, processing of suppressing the intensity (the volume) of a signal other than voice output from the external equipment 120. In the present application example, the external equipment 120 may change content of processing depending on whether received information is actual measurement information or prediction information. For example, when the received information is the prediction information, it is conceivable to reduce an effect of the processing compared with when the received information is the measurement information.

As a second example, the external equipment 120 can suppress, for example, for the subject 901 having a strong degree of the sensorineural hearing loss, sound of a powerfully closed door and sudden sound such as a gunshot. In the case of the sensorineural hearing loss, compression processing is usually performed by the hearing aid 116 because of a replenishment phenomenon. However, an attack time (specifically, a time from a rise of sound until a compression amount reaches a predetermined value) cannot be reduced much in order to keep naturalness of intonation of voice. Therefore, in order to suppress sudden sound without reducing the attack time, it is effective to read ahead an acoustic signal.

FIG. 30 illustrates an example of an effect of the suppression of sudden sound and the read-ahead. The top part of FIG. 30 illustrates an example in which weak sound suddenly changes to strong sound. Specifically, in the example, the weak sound suddenly changes to strong sound at time of 20 msec. A second part from the top in FIG. 30 is an example in which sudden noise is suppressed by compression in a case without the read-ahead (without a processing delay). The sudden sound is sufficiently suppressed around time of 40 msec but does not change much around 20 msec from the example of the top part of FIG. 30. The bottom part of FIG. 30 illustrates an example in which read-ahead of 4 msec (that is, with a processing delay of 4 msec) is allowed. As illustrated in the bottom part of FIG. 30, it is seen that the sudden sound is also suppressed around time of 24 msec. Note that a suppression amount can be further increased by further increasing the length of the read-ahead.

However, the read-ahead in the hearing aid 116 has a serious adverse effect. Since the read-ahead causes a processing delay, the hearing aid 116 cannot actively use the read-ahead. When the processing delay occurs, there is a problem in that the processing delay leads to discomfort of mixing of sound entering from a gap between the hearing aid 116 and the ear canal and sound amplified by the hearing aid 116 or discomfort to a time difference between visual sensation and auditory sensation.

However, although the read-ahead is not a realistic choice in the hearing aid 116, the read-ahead is considered to be a realistic and effective choice in the external equipment 120. Thus, a block diagram of the external equipment 120 having such a configuration for audio video control is illustrated in FIG. 31. As illustrated in FIG. 31, the external equipment 120 mainly includes an audio video control unit 595, a hearing information storage unit 596, an audio processing unit 597, and a video delay amount adjustment unit 598.

The hearing information storage unit 596 stores the hearing information such as the degree prediction information of the conductive hearing loss of the subject 901, the air conduction hearing level, and the parameter transmitted from the information processing terminal 117 or the like. The audio video control unit 595 controls the audio processing unit 597 based on the hearing information stored in the hearing information storage unit 596. For example, the audio processing unit 597 processes an audio signal illustrated in the top part of FIG. 30 to be an audio signal illustrated in the bottom part of FIG. 30 and outputs the audio signal. The audio signal processed by the audio processing unit 597 has a delay. Thus, the audio video control unit 595 controls the video delay amount adjustment unit 598. Specifically, the video delay amount adjustment unit 598 receives the video signal, adjusts a delay amount, and outputs the video signal. The delay amount added by video delay amount adjustment unit 598 is determined depending on the delay amount in the audio processing unit 597.

As explained above, according to the present application example, by adjusting the delay amount of the video signal depending on the processing delay of the audio signal, it is possible to suppress sudden sound of the audio signal while suppressing deviation between the audio signal and the video signal.

In addition, audio processing performed by the audio processing unit 597 may be processing of supplementing the hearing level of the subject 901 based on the hearing information stored in the hearing information storage unit 596 or may be processing of obtaining an effect different from the hearing level. For example, when the user of the hearing aid 116 is listening to sound output from a speaker of the television device through the hearing aid 116, processing of giving a delay such as sudden sound suppression may be performed on the television device side and processing of supplementing the hearing level may be left to normal processing on the hearing aid 116 side. Accordingly, when the hearing aid user and his/her family are watching television together, the family can also enjoy natural sound while suppressing discomfort due to sudden sound of the hearing aid user. As explained above, since the hearing aid 116 cooperates with the external equipment 120, it is possible to supplement a part that the hearing aid 116 is not good at.

Note that, in the present application example, the functional configuration of the external equipment 120 is not limited to the configuration illustrated in FIG. 31.

<<6. Summary>>

As explained above, in the embodiment of the present disclosure, the degree of the conductive hearing loss can be predicted using the air conduction headset, that is, the air conduction sound. As a result, according to the embodiment of the present disclosure, it is possible to recommend the subject 901 to consult a medical institution or the like and it is possible to prevent the subject 901 from missing an opportunity to early treat a disorder causing a hearing loss. Further, according to the embodiments of the present disclosure, a gain of the hearing aid 116 of the subject 901 can be appropriately set according to the hearing of the subject 901.

<<7. Overview of hearing aid system>>

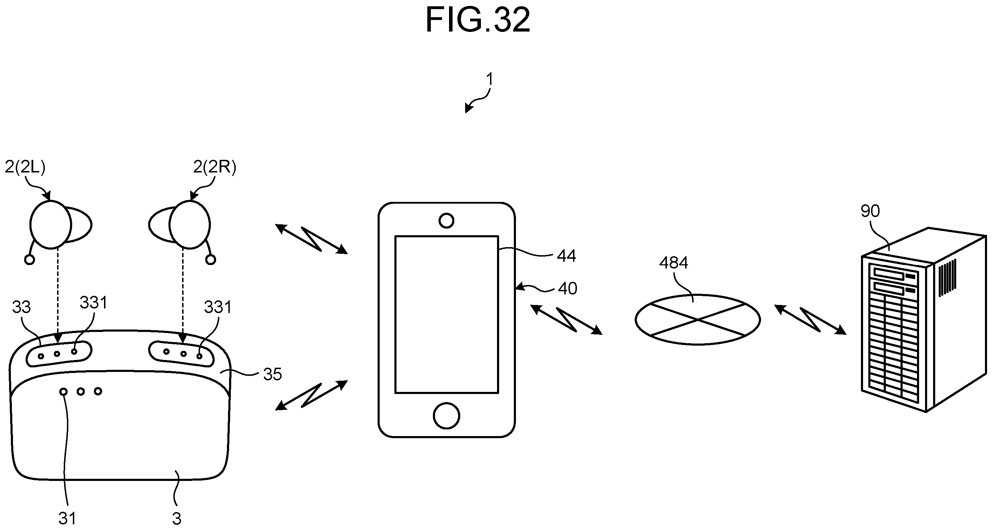

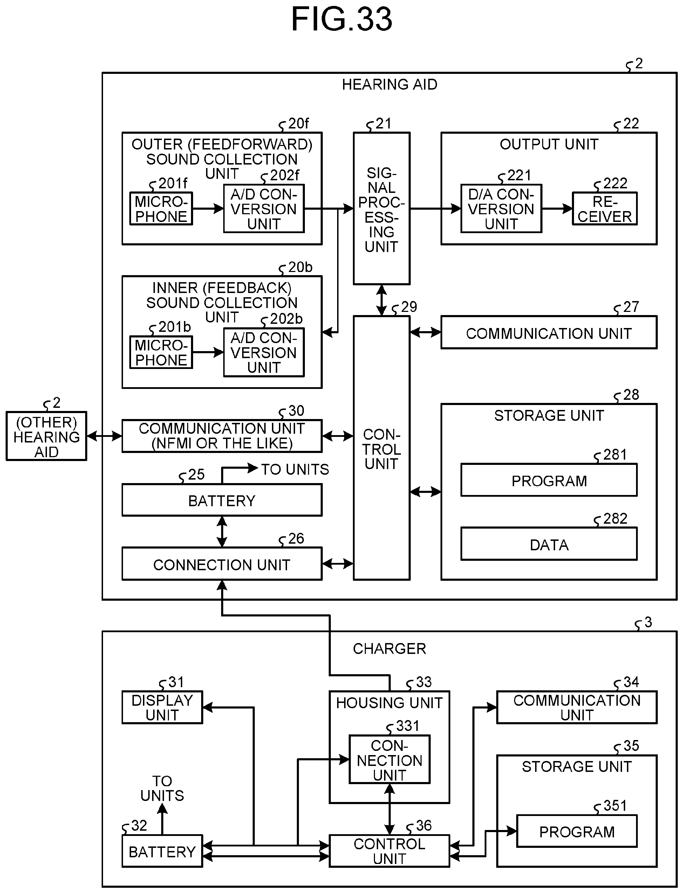

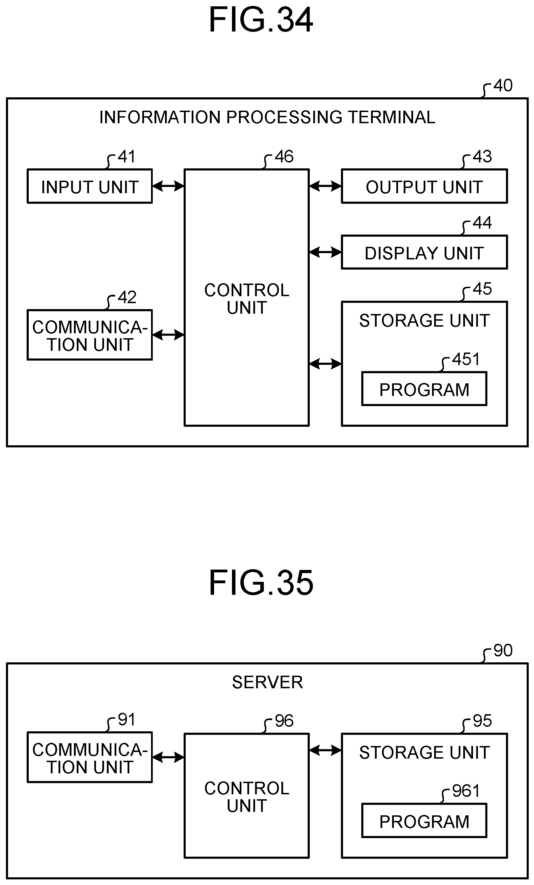

An overview of a hearing aid system 1 according to an embodiment of the present disclosure is explained with reference to FIG. 32 to FIG. 35. FIG. 32 is a diagram illustrating a schematic configuration of the hearing aid system 1 according to the embodiment of the present disclosure, FIG. 33 is a functional block diagram of a hearing aid 2 and a charger 3 according to the embodiment of the present disclosure, and FIG. 34 is a block diagram of an information processing terminal 40 according to the embodiment of the present disclosure. Further, FIG. 35 is a block diagram of a server 90 according to the embodiment of the present disclosure.

As illustrated in FIG. 32, a hearing aid system 1 according to an embodiment of the present disclosure includes a pair of left and right hearing aids 2, a charger 3 (a charging case) that houses the hearing aids 2 and charges the hearing aids 2, and an information processing terminal 40 such as a smartphone capable of communicating with at least one of the hearing aids 2 and the charger 3. Further, the hearing aid system 1 according to the embodiment of the present disclosure includes a server 90 managed by the hearing aid sales company 291 or the auditory test service providing company 292. The devices included in the hearing aid system 1 according to the embodiment of the present disclosure are explained below in order.

In the following explanation, it is assumed that the hearing aid 2 is configured by a pair of hearing aids for both ears. However, the embodiment of the present disclosure is not limited to this and may be a single-ear type hearing aid worn on one of the left and right ears.

First, a functional configuration of the hearing aid 2 is explained. In an embodiment of the present disclosure, at least a part of the hearing aid 2 is configured to be worn on a part of the ear canal of a user. Then, as illustrated in FIG. 33, the hearing aid 2 mainly includes sound collection units 20 (20b, 20f), a signal processing unit 21, an output unit 22, a battery 25, a connection unit 26, communication units 27 and 30, a storage unit 28, and a control unit 29.

The sound collection unit 20 includes an outer (feedforward) sound collection unit 20f that collects sound in an outer region of the ear canal and an inner (feedback) sound collection unit 20b that collects sound in an inner region of the ear canal. Note that, in the hearing aid 2 according to the embodiment of the present disclosure, an outer sound collection unit 20f that collects at least sound in the outer region of the ear canal only has to be provided. The sound collection units 20 include microphones (hereinafter also referred to as microphones)201 and analog/digital (A/D) conversion units 202. The microphones 201 collect sound, generate analog audio signals (acoustic signals), and output the analog audio signals to the A/D conversion unit 202. The A/D conversion unit 202 perform digital conversion processing on the analog audio signals input from the microphones 201 and output the digitized audio signals to the signal processing unit 21.

Under control of the control unit 29 explained below, the signal processing unit 21 performs predetermined signal processing on the digital audio signals input from the sound collection units 20 and outputs the digital audio signals to the output unit 22. Here, examples of the predetermined signal processing include filtering processing of separating an audio signal for each predetermined frequency band, amplification processing of amplifying the audio signal with a predetermined amplification amount for each predetermined frequency band on which the filtering processing has been performed, noise reduction processing, and howling cancellation processing. The signal processing unit 21 can be configured by, for example, a memory and a processor including hardware such as a DSP (Digital Signal Processor).

The output unit 22 includes a D/A (digital/analog) conversion unit 221 and a receiver 222. The D/A conversion unit 221 performs analog conversion processing on the digital audio signals input from the signal processing unit 21 and outputs the digital audio signals to the receiver 222. The receiver 222 outputs output sound (voice) corresponding to the analog audio signals input from the D/A conversion unit 221. The receiver 222 can be configured using, for example, a speaker.

The battery 25 supplies electric power to the units configuring the hearing aid 2. The battery 25 can be configured by, for example, a rechargeable secondary battery such as a lithium ion battery. Further, the battery 25 can be charged by electric power supplied from the charger 3 via the connection unit 26.

For example, when the hearing aid 2 is housed in the charger 3, the connection unit 26 is connected to the connection unit of the charger 3 and can receive electric power and various kinds of information from the charger 3 and output various kinds of information to the charger 3. The connection unit 26 can be configured using, for example, a plurality of pins.

The communication unit 27 can communicate with the charger 3 or the information processing terminal 40 according to a predetermined communication standard via the Internet 484 under the control of the control unit 29. Here, as the predetermined communication standard, for example, Wi-Fi (registered trademark), Bluetooth (registered trademark), and the like are assumed. The communication unit 27 can be configured using, for example, a communication module or the like. Further, under the control of the control unit 29, the communication unit 30 can communicate with the other hearing aid 2 by short-range communication such as NFMI (Near Field Magnetic Induction).

The storage unit 28 stores various kinds of information concerning the hearing aid 2. The storage unit 28 can be configured using, for example, a RAM (Random Access Memory), a ROM (Read Only Memory), a memory card, or the like. The storage unit 28 can store a program 281 to be executed by the hearing aid 2 and various data 282 used in the hearing aid 2. Examples of the data 282 include the age of the user, the presence or absence of use experience of the hearing aid 2 of the user, and the sex of the user. Further, examples of the data include a use time of use of the hearing aid 2 by the user clocked by a clocking unit (not illustrated). In addition, the clocking unit is provided inside the hearing aid 2 and can measure a date and time and output a clocking result to the control unit 29 and the like. The clocking unit can be configured using, for example, a timing generator or a timer having a clocking function.

The control unit 29 controls the units configuring the hearing aid 2. The control unit 29 can be configured using, for example, a memory and a processor including hardware such as a central processing unit (CPU) or a DSP. The control unit 29 reads out the stored program 281 to a work area of the memory and controls the components and the like through execution of the program by the processor.

Although not illustrated in FIG. 33, the hearing aid 2 may include an operation unit. The operation unit can receive input of a start signal (a trigger signal) for starting the hearing aid 2 and output the received start signal to the control unit 29. The operation unit can be configured using, for example, a push switch, a button, or a touch panel.

Subsequently, a functional configuration of the charger 3 is explained. As illustrated in FIG. 33, the charger 3 mainly includes a display unit 31, a battery 32, a housing unit 33, a communication unit 34, a storage unit 35, and a control unit 36.

The display unit 31 displays various states concerning the hearing aid 2 under the control of the control unit 36. For example, the display unit 31 can display information indicating that the hearing aid 2 is being charged and information indicating that various kinds of information are being received from the information processing terminal 40. The display unit 31 can be configured using, for example, a light emitting diode (LED).

The battery 32 supplies electric power to the units configuring the hearing aid 2 and the charger 3 housed in the housing unit 33 via a connection unit 331 provided in the housing unit 33. The battery 32 can be configured using a secondary battery such as a lithium ion battery.

The housing unit 33 individually stores each of the left and right hearing aids 2. In the housing unit 33, the connection unit 331 connectable to the connection unit 26 of the hearing aid 2 is provided. When the hearing aid 2 is housed in the housing unit 33, the connection unit 331 is connected to the connection unit 26 of the hearing aid 2, transmits electric power from the battery 32 and transmits various kinds of information from the control unit 36, receives various kinds of information from the hearing aid 2, and outputs the information to the control unit 36. The connection unit 331 can be configured using, for example, a plurality of pins.

The communication unit 34 communicates with the information processing terminal 40 according to a predetermined communication standard via a communication network under the control of the control unit 36. The communication unit 34 can be configured using, for example, a communication module.

The storage unit 35 stores various programs 351 to be executed by the charger 3. The storage unit 35 can be configured using, for example, a RAM, a ROM, a flash memory, or a memory card.

The control unit 36 controls the units configuring the charger 3. For example, when the hearing aid 2 is housed in the housing unit 33, the control unit 36 causes the battery 32 to supply electric power via the connection unit 331. The control unit 36 can be configured using, for example, a memory and a processor including hardware such as a CPU or a DSP. The control unit 36 reads out the programs 351 to a work area of the memory and executes the programs 351 and controls the components and the like through the execution of the programs by the processor.

Subsequently, a functional configuration of the information processing terminal 40 is explained. As illustrated in FIG. 34, the information processing terminal 40 mainly includes an input unit 41, a communication unit 42, an output unit 43, a display unit 44, a storage unit 45, and a control unit 46.

The input unit 41 receives input of various kinds of operation from the user and outputs a signal corresponding to the received operation to the control unit 46. The input unit 41 can be configured using, for example, a switch or a touch panel.

The communication unit 42 communicates with the charger 3 or the hearing aid 2 via a communication network under the control of the control unit 46. The communication unit 42 can be configured using, for example, a communication module.

The output unit 43 outputs a sound volume of a predetermined sound pressure level for each predetermined frequency band under the control of the control unit 46. The output unit 43 can be configured using, for example, a speaker.

The display unit 44 displays various kinds of information concerning the information processing terminal 40 and information concerning the hearing aid 2 under the control of the control unit 46. The display unit 44 can be configured using, for example, a liquid crystal display or an organic EL display (Organic Electroluminescent Display).

The storage unit 45 stores various kinds of information concerning the information processing terminal 40. The storage unit 45 stores, for example, various programs 451 to be executed by the information processing terminal 40. The storage unit 45 can be configured using, for example, a recording medium such as a RAM, a ROM, a flash memory, or a memory card.

The control unit 46 controls the units configuring the information processing terminal 40. The control unit 46 can be configured using, for example, a memory and a processor including hardware such as a CPU. The control unit 46 reads out the programs stored in the storage unit 45 to a work area of the memory and executes the programs and controls the components and the like through execution of the programs by the processor.

Further, the server 90 may be configured as illustrated in FIG. 35. As illustrated in FIG. 35, the server 90 mainly includes a communication unit 91, a storage unit 95, and a control unit 96.

The communication unit 91 communicates with the hearing aid 2 and the information processing terminal 40 via the Internet 484 under the control of the control unit 96. The communication unit 91 can be configured using, for example, a communication module. The storage unit 95 stores various kinds of information concerning the hearing aid 2. Further, the storage unit 95 stores, for example, various programs 961 to be executed by the server 90. The storage unit 95 can be configured using, for example, a recording medium such as a RAM, a ROM, a flash memory, or a memory card.

The control unit 96 controls the units configuring the server 90. The control unit 96 can be configured using, for example, a memory and a processor including hardware such as a CPU. The control unit 96 reads out the programs stored in the storage unit 95 to a work area of the memory and executes the programs and controls the components and the like through execution of the programs by the processor.

Note that, in the embodiment of the present disclosure, the functional configurations of the hearing aid system 1 and the devices included in the hearing aid system 1 are not limited to the forms illustrated in FIG. 32 to FIG. 35.

<<8. Example of utilization of data>>

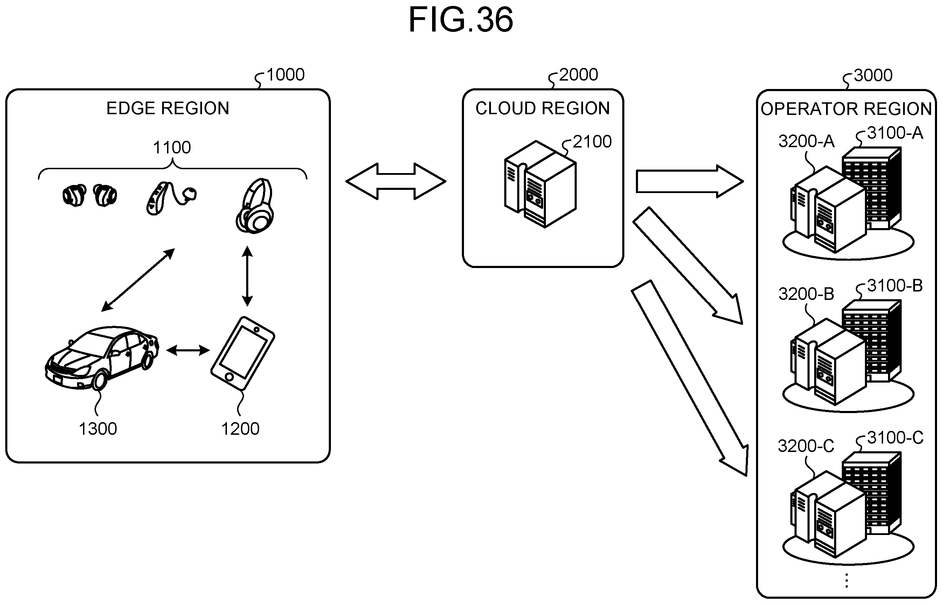

Data obtained in relation to utilization of a hearing aid device may be utilized in various ways. An example is explained with reference to FIG. 36.

FIG. 36 is a diagram illustrating an example of utilization of data. In an exemplified system, an edge region 1000, a cloud region 2000, and an operator region 3000 are present. As elements in the edge region 1000, a sound emitting device 1100, a peripheral device 1200, and a vehicle 1300 are exemplified. As an element in the cloud region 2000, a server device 2100 is exemplified. As elements in the operator region 3000, an operator 3100 and a server device 3200 are exemplified.

The sound emitting device 1100 in the edge region 1000 is used by being worn by the user or disposed near the user to emit sound toward the user. Specific examples of the sound emitting device 1100 include an earphone speaker, a headset (a headphone speaker), and a hearing aid. More specifically, the sound emitting device 1100 can be the headphone speaker 115 in FIG. 16 or the hearing aid 2 in FIG. 32.

The peripheral device 1200 and the vehicle 1300 in the edge region 1000 are devices used together with the sound emitting device 1100 and transmit signals of, for example, content viewing sound and speech sound to the sound emitting device 1100. The sound emitting device 1100 outputs sound corresponding to a signal from the peripheral device 1200 or the vehicle 1300 to the user. A specific example of the peripheral device 1200 is a smartphone or the like.

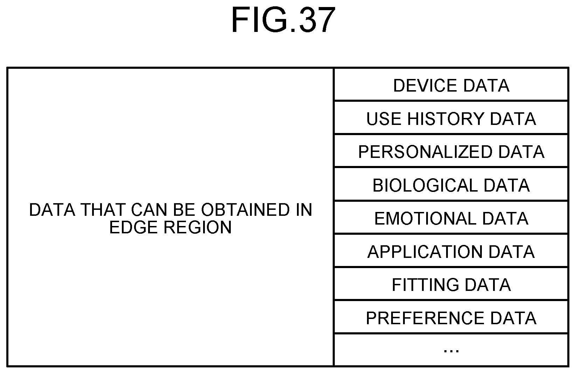

Within the edge region 1000, various data concerning utilization of the sound emitting device 1100 can be obtained. FIG. 37 is also referred to for the explanation.

FIG. 37 is a diagram illustrating an example of data. Examples of data that can be acquired in the edge region 1000 include device data, use history data, personalized data, biological data, emotional data, application data, fitting data, and preference data. Note that the data may be understood as meaning of information and may be read as appropriate as long as there is no contradiction. Various publicly known methods may be used to acquire the exemplified data.

The device data is data concerning the sound emitting device 1100 and includes, for example, type data of the sound emitting device 1100, specifically, data for specifying that the sound emitting device 1100 is an earphone, a headphone, a TWS (True Wireless Stereo), a hearing aid (CIC (Completely In-The-Canal), ITE (In-The-Ear), RIC (Receiver-In-The-Canal), and the like), or the like.

The use history data is use history data of the sound emitting device 1100 and includes, for example, data such as a music exposure dose, a continuous use time of a hearing aid, and a content viewing history (a viewing time and the like). Further, the use history data may also include a use time, the number of times of use, and the like of a function such as transmission of an utterance flag in the embodiment explained above. The use history data can be used for safe listening, conversion of TWS into a hearing aid, replacement notification of a wax guard (a cerumen intrusion preventing filter), and the like.

The personalized data is data concerning a user of the sound emitting device 1100 and includes, for example, a head related transfer function (HRTF) of an individual user, an air conduction hearing level, and a type of earwax. Further, data such as hearing may also be included in the personalized data.

The biological data is biological data of the user of the sound emitting device 1100 and includes data such as perspiration, a blood pressure, a blood flow, a heart rate, a pulse, a body temperature, an electroencephalogram, a respiration, and a myoelectric potential.

The emotional data is data indicating emotion of the user of the sound emitting device 1100 and includes, for example, data indicating comfort, discomfort, and the like.

The application data is data, for example, used in various applications and includes, for example, user attribute information data such as a position of the user of the sound emitting device 1100 (or a position of the sound emitting device 1100) and a schedule, age, and sex of the user and data such as weather, atmospheric pressure, and temperature. For example, the position data can be used to search for a missing sound emitting device 1100.

The fitting data can include, for example, adjustment parameters of the hearing aid 2 or the headphone speaker 115 used by the user and a hearing aid gain for each frequency band set based on a hearing measurement result (an audiogram) of the user.

The preference data is data concerning preference of the user and includes data such as preference of music to listen to during driving.

Note that data of a communication status, data of a charging status of the sound emitting device 1100, and the like may also be acquired. A part of the processing in the edge region 1000 may be executed by the cloud region 2000 according to a band, a communication status, a charging status, and the like. Since the processing is shared, a processing load in the edge region 1000 is reduced.

Referring back to FIG. 36, for example, the data as explained above is acquired in the edge region 1000 and transmitted from the sound emitting device 1100, the peripheral device 1200, or the vehicle 1300 to the server device 2100 in the cloud region 2000. The server device 2100 stores (for example, saves or accumulates) the received data.

The operator 3100 in the operator region 3000 uses the server device 3200 to acquire data from the server device 2100 in the cloud region 2000. The data can be utilized by the operator 3100.

Various operators 3100 can be present. Specific examples of the operator 3100 include a hearing aid store, a hearing aid manufacturer, a content production company, and a distribution operator providing a music streaming service and the like. These operators are referred to and illustrated as operator 3100-A, operator 3100-B, and operator 3100-C to be distinguishable. Server devices 3200 corresponding to the operators are referred to and illustrated as server device 3200-A, server device 3200-B, and server device 3200-C. The various data are provided to such various operators 3100 and utilization of the data is promoted. The data provision to the operator 3100 may be data provision by subscription, recalling, or the like.

Data provision from the cloud region 2000 to the edge region 1000 is also possible. For example, when machine learning is necessary to implement processing in the edge region 1000, data for feedback, correction (Revise), and the like of learning data is prepared by an administrator or the like of the server device 2100 in the cloud region 2000. The prepared data is transmitted from the server device 2100 to the sound emitting device 1100, the peripheral device 1200, or the vehicle 1300 in the edge region 1000.

When a specific condition is satisfied in the edge region 1000, some incentive (a privilege such as a premium service) may be provided to the user. An example of the condition is a condition that at least a part of the sound emitting device 1100, the peripheral device 1200, and the vehicle 1300 are devices provided by the same operator. If the incentive is an incentive (an electronic coupon or the like) that can be electronically supplied, the incentive may be transmitted from the server device 2100 to the sound emitting device 1100, the peripheral device 1200, or the vehicle 1300.

<<9. Example of cooperation with other devices>>

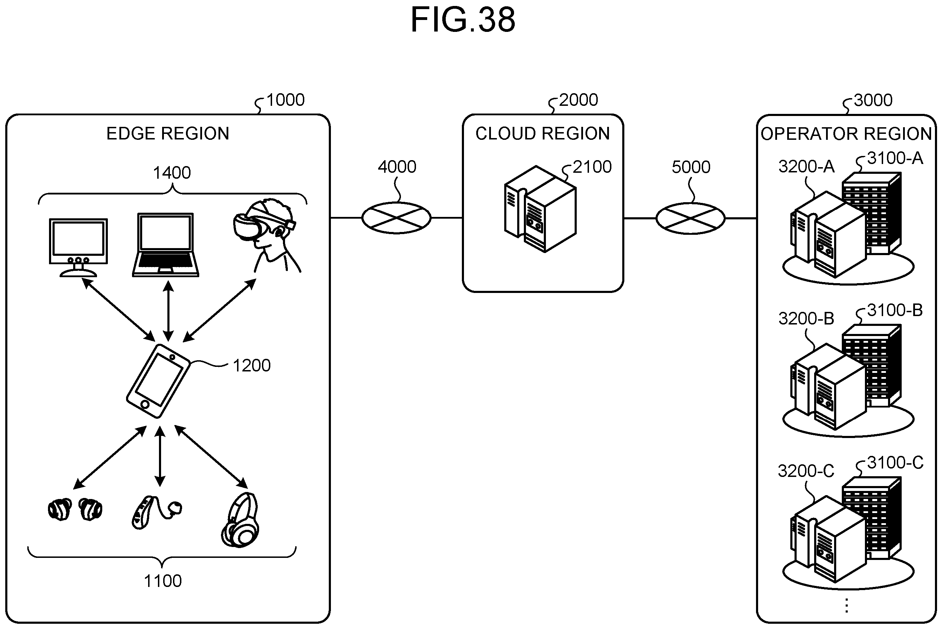

In the edge region 1000, for example, the sound emitting device 1100 and other devices may cooperate using the peripheral device 1200 such as a smartphone as a hub. An example is explained with reference to FIG. 38.

FIG. 38 is a diagram illustrating an example of cooperation with other devices. The edge region 1000, the cloud region 2000, and the operator region 3000 are connected by a network 4000 and a network 5000. A smartphone is exemplified as the peripheral device 1200 in the edge region 1000 and other devices 1400 are also exemplified as elements in the edge region 1000. Note that illustration of the vehicle 1300 (FIG. 36) is omitted.

The peripheral device 1200 is capable of communicating with each of the sound emitting device 1100 and the other devices 1400. A communication method is not particularly limited but, for example, Bluetooth LDAC, Bluetooth LE Audio explained above, or the like may be used. Communication between the peripheral device 1200 and the other devices 1400 may be multicast communication. Examples of the multicast communication are Auracast (registered trademark) and the like.

The other devices 1400 are used in cooperation with the sound emitting device 1100 via the peripheral device 1200. Specific examples of the other device 1400 include a television (hereinafter referred to as television), a personal computer (PC), a HMD (Head Mounted Display), a robot, a smart speaker, and a gaming device.

Even in a case in which the sound emitting device 1100, the peripheral device 1200, and the other device 1400 satisfy a specific condition (For example, a condition that at least a part thereof is provided by the same operator.), an incentive may be provided to the user.

The sound emitting device 1100 and the other device 1400 are capable of cooperating using the peripheral device 1200 as a hub. The cooperation may be performed using various data stored in the server device 2100 in the cloud region 2000. For example, information such as fitting data, a viewing time, and hearing of the user is shared between the sound emitting device 1100 and the other devices 1400, whereby volume adjustment and the like of the device are performed in cooperation. When the hearing aid 2 (HA) or a sound collector (PSAP: Personal Sound Amplification Product) is worn, setting for the hearing aid 2 or the PSAP can be automatically performed on a television, a PC, or the like. For example, when the user using the hearing aid 2 uses another device such as a television or a PC, processing of automatically changing setting of the other device may be performed such that usual setting for a normal listener changes to setting suitable for the user using the hearing aid. Note that whether the user is using the hearing aid 2 may be determined by, when the user wears the hearing aid 2, information indicating that the user has worn the hearing aid 2 (for example, wearing detection information) being automatically sent to equipment such as a television or a PC at a pairing destination of the hearing aid 2 or may be detected while being triggered by the user using the hearing aid approaching another device such as a television or a PC set as a target. By imaging the face of the user with a camera or the like provided in another device such as a television or a PC or by a method other than the method explained above, it may be determined that the user is a hearing aid user. It is also possible to cause the hearing aid 2 to function as an earphone by, for example, the hearing aid 2, which is the sound emitting device 1100, and the other devices 1400 cooperating with each other. Further, when the other devices 1400 include microphones that collect ambient sound, it is also possible to cause an earphone, which is the sound emitting device 1100, to function like the hearing aid 2. In this case, the function of the hearing aid can be used in a style (appearance or the like) as if listening to music. The earphone or the headphone and the hearing aid have many technically overlapping portions. It is assumed that a barrier between the earphone or the headphone and the hearing aid disappears in the future and one device has functions of both of the earphone and the hearing aid. When the hearing is normal, that is, for a normal listener, it is possible to enjoy a content viewing experience by using the hearing aid as a usual earphone or headphone. When the hearing decreases because of aging or the like, the hearing aid can function as the hearing aid by turning on the hearing aid function. Since the device as the earphone can be directly used as a hearing aid, continuous and long-term use by the user can be expected from the viewpoint of appearance and design as well.

Data of a listening history of the user may be shared. Listening for a long time can be a risk for a hearing loss in the future. Notification or the like to the user may be performed such that the listening time does not become too long. For example, when the viewing time exceeds a predetermined threshold, such a notification is performed (safe listening). The notification may be performed by any device in the edge region 1000.

At least a part of the devices used in the edge region 1000 may be provided by different operators. Information concerning device settings and the like of the operators may be transmitted from the server devices 3200 in the operator region 3000 to the server device 2100 in the cloud region 2000 and stored in the server device 2100. By using such information, the devices provided by the different operators are also capable of cooperating with one another.

<<10. Example of use transition>>

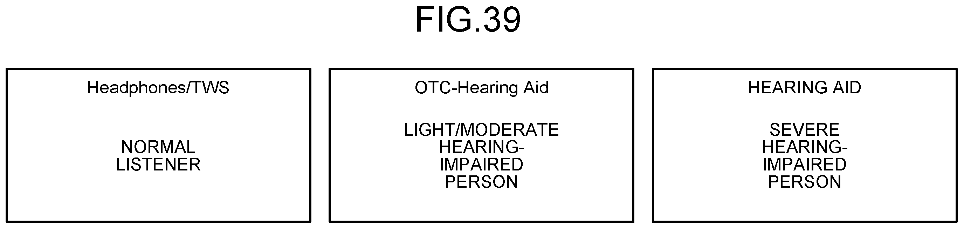

A use of the sound emitting device 1100 can transition according to various situations including the fitting data, the viewing time, and the hearing of the user explained above. An example is explained with reference to FIG. 39.

FIG. 39 is a diagram illustrating an example of use transition. When the user is a normal listener, for example, while the user is a child and for a while after the user becomes an adult, the sound emitting device 1100 is used as a headphone or an earphone (headphones/TWS). Besides the safe listening explained above, adjustment of an equalizer and processing (for example, a noise cancelling mode is switched to optimum noise canceling modes respectively for a scene in which the user is at a restaurant and a scene in which the user is riding on a vehicle) corresponding to a behavior characteristic and a current location of the user and an external environment are performed or viewing music log collection and the like are performed. Communication between devices using Auracast is also used.

When hearing of the user is deteriorated, the hearing aid function of the sound emitting device 1100 is started to be utilized. For example, while the user is a light or moderate hearing-impaired person, the sound emitting device 1100 is used as an OTC hearing aid. When the user is a severe hearing-impaired person, the sound emitting device 1100 is used as a hearing aid. Note that the OTC hearing aid is a hearing aid that is sold at a store without intervention of an expert and has easiness of being able to be purchased not through a hearing test or an expert such as an audiologist. Operation specific to the hearing aid such as fitting may be performed by the user himself/herself. While the sound emitting device 1100 is used as the OCT hearing aid or the hearing aid, hearing measurement is performed or the hearing aid function is turned on. For example, the function such as the transmission of the utterance flag in the embodiment explained above can also be used. Various kinds of information concerning hearing (hearing big data) are collected, fitting, sound environment adaptation, remote support, and the like are performed and a transcription is further performed.

<<11. Supplement>>

Note that the embodiments of the present disclosure explained above can include, for example, an information processing method executed by the information processing device or the information processing system explained above, a program for causing the information processing device (a computer) to function, and a non-transitory tangible medium in which the program is recorded. The program may be distributed via a communication line (including wireless communication) such as the Internet.

The steps in the information processing method in the embodiment of the present disclosure explained above may not always be processed according to the described order. For example, the steps may be processed with the order changed as appropriate. The steps may be partially processed in parallel or individually instead of being processed in time series. Further, the processing of the steps may not always be processed according to the described method and may be processed by, for example, another functional unit according to another method.

Among the kinds of processing explained in the embodiments explained above, all or a part of the processing explained as being automatically performed can be manually performed or all or a part of the processing explained as being manually performed can be automatically performed by a publicly-known method. Besides, the processing procedures, the specific names, and the information including the various data and parameters explained in the document and illustrated in the drawings can be optionally changed except when specifically noted otherwise. For example, the various kinds of information illustrated in the figures are not limited to the illustrated information.

The illustrated components of the devices are functionally conceptual and are not always required to be physically configured as illustrated in the figures. That is, specific forms of distribution and integration of the devices are not limited to the illustrated forms and all or a part thereof can be configured by being functionally or physically distributed and integrated in any unit according to various loads, usage situations, and the like.

The preferred embodiment of the present disclosure is explained in detail above with reference to the accompanying drawings. However, the technical scope of the present disclosure is not limited to such an example. It is evident that those having the ordinary knowledge in the technical field of the present disclosure can arrive at various alterations or corrections within the category of the technical idea described in claims. It is understood that these alterations and corrections naturally belong to the technical scope of the present disclosure.

The effects described in the present specification are only explanatory or illustrative and are not limiting. That is, the technique according to the present disclosure can achieve other effects obvious for those skilled in the art from the description of the present specification together with or instead of the effects explained above.

Note that the present technique can also take the following configurations.

(1) An information processing device comprising
a prediction unit that predicts a degree of a conductive hearing loss based on test results of an auditory test using air conduction sound, the auditory test being first and second auditory tests including test contents different from each other.

(2) The information processing device according to (1), wherein

the test result of the first auditory test includes an air conduction auditory threshold, and
the test result of the second auditory test includes a test result about an inner ear and/or a posterior labyrinthine.

(3) The information processing device according to (1) or (2), wherein the prediction unit predicts the degree of the conductive hearing loss using a statistical method.

(4) The information processing device according to (1) or (2), wherein the prediction unit predicts the degree of the conductive hearing loss using a learned model.

(5) The information processing device according to any one of (1) to (4), wherein the first auditory test is an air conduction hearing test and/or a self-recording audiometry by intermittent sound.

(6) The information processing device according to any one of (1) to (5), wherein

the second auditory test
includes at least one test selected out of a group consisting of a self-recording audiometry by continuous sound, an SISI test, an ABLB test, a DL test, a TD test, a speech recognition threshold test, a highest speech intelligibility test, a distorted speech hearing test, a binaural separation function test, and a sense of direction test.

(7) The information processing device according to any one of (1) to (4), wherein

the first auditory test is a self-recording audiometry by intermittent sound, and
the second auditory test is a self-recording audiometry by continuous sound.

(8) The information processing device according to any one of (1) to (5), wherein
the prediction unit predicts the degree of conductive hearing loss based on attribute information of a subject.

(9) The information processing device according to any one of (1) to (8), further comprising
a sound source generation unit that generates the air conduction sound used in the first and second auditory tests.

(10) The information processing device according to any one of (1) to (9), further comprising
a correction unit that acquires output characteristic information of a sound output device, which outputs the generated air conduction sound, and corrects the generated air conduction sound based on the acquired output characteristic information.

(11) The information processing device according to (10), wherein the correction unit acquires the output characteristic information based on an image of the sound output device.

(12) The information processing device according to any one of (1) to (11), further comprising an output unit that outputs the test result of the first auditory test, the test result of the second auditory test, and the predicted degree of the conductive hearing loss.

(13) The information processing device according to any one of (1) to (12), further comprising an output unit that compares the predicted degree of the conductive hearing loss and a predetermined threshold and outputs, based on a comparison result, information for urging a subject to consult a medical institution.

(14) The information processing device according to any one of (1) to (12), further comprising an output unit that compares the predicted degree of the conductive hearing loss and a predetermined threshold and outputs, based on a comparison result, information for purchasing a hearing aid to a subject.

(15) The information processing device according to any one of (1) to (14), further comprising a parameter determination unit that determines a setting parameter of a hearing aid based on the predicted degree of the conductive hearing loss.

(16) The information processing device according to (15), wherein the setting parameter is a parameter for performing gain setting control and/or noise suppression setting control for the hearing aid.

(17) The information processing device according to any one of (1) to (14), further comprising a parameter determination unit that determines, based on the predicted degree of the conductive hearing loss, a setting parameter of an acoustic device outside the information processing device.

(18) The information processing device according to (17), wherein the setting parameter is a parameter for delaying voice output from the acoustic device or controlling volume of the voice output from the acoustic device.

(19) An information processing method comprising predicting, by an information processing device, a degree of a conductive hearing loss based on test results of an auditory test using air conduction sound, the auditory test being first and second auditory tests including test contents different from each other.

(20) A program for causing a computer to execute a function of predicting a degree of a conductive hearing loss based on test results of an auditory test using air conduction sound, the auditory test being first and second auditory tests including test contents different from each other.

(21) A learned model generation method including inputting, to a learning device, test results of an auditory test using air conduction sound, the auditory test being first and second auditory tests including test contents different from each other, as input data and inputting a degree of a conductive hearing loss corresponding to the test results of the first and second auditory tests as teacher data, wherein
the learning device generates a learned model for predicting the degree of the conductive hearing loss based on the test results of the first and second auditory tests.

Reference Signs List

1 HEARING AID SYSTEM
2, 116 HEARING AID
3 CHARGER
20b, 20f SOUND COLLECTION UNIT
21 SIGNAL PROCESSING UNIT
22 OUTPUT UNIT
25, 32 BATTERY
26, 331 CONNECTION UNIT
27, 30, 34, 42, 91 COMMUNICATION UNIT
28, 35, 45, 95 STORAGE UNIT
29, 36, 46, 96 CONTROL UNIT
31, 44 DISPLAY UNIT
33 HOUSING UNIT

40, 117 INFORMATION PROCESSING TERMINAL
41 INPUT UNIT
43 OUTPUT UNIT
90 SERVER
102, 901 SUBJECT
115 HEADPHONE SPEAKER
119, 484 INTERNET
120 EXTERNAL EQUIPMENT
121 FIRST PATH
122 SECOND PATH
161 FIRST GROUP AUDITORY TEST SOUND SOURCE GENERATION UNIT
162 SECOND GROUP AUDITORY TEST SOUND SOURCE GENERATION UNIT
163 TEST CONTROL UNIT
164 CONDUCTIVE HEARING LOSS DEGREE PREDICTION UNIT
165 TEST INFORMATION STORAGE UNIT
166 ADDITIONAL INFORMATION STORAGE UNIT
171 AUDITORY TEST SOUND OUTPUT MEANS
172 INFORMATION OUTPUT MEANS
173 INFORMATION INPUT MEANS
174 COMMUNICATION MEANS
189 LEVEL/FREQUENCY CHARACTERISTIC CORRECTION INFORMATION STORAGE UNIT
190 LEVEL/FREQUENCY CHARACTERISTIC CORRECTION UNIT
197 HEARING AID PARAMETER DETERMINATION UNIT
198 HEARING AID PARAMETER STORAGE UNIT
201b, 201f MICROPHONE
202b, 202f A/D CONVERSION UNIT
221 D/A CONVERSION UNIT
222 RECEIVER
281, 351 PROGRAM
282 DATA
291 HEARING AID SALES COMPANY
292 AUDITORY TEST SERVICE PROVIDING COMPANY
299 DATABASE
333, 334, 335, 336, 337, 338, 541, 542, 543, 544, 545, 546 CORRESPONDING RANGE
595 AUDIO VIDEO CONTROL UNIT
596 HEARING INFORMATION STORAGE UNIT
597 AUDIO PROCESSING UNIT
598 VIDEO DELAY AMOUNT ADJUSTMENT UNIT
751 FIRST SECTION
752 SECOND SECTION
753 THIRD SECTION
754 FOURTH SECTION
755 FIFTH SECTION
756 SIXTH SECTION
757 SEVENTH SECTION
758 EIGHTH SECTION
759 NINTH SECTION
781 AUDITORY TEST INFORMATION OF FIRST GROUP
782 AUDITORY TEST INFORMATION OF SECOND GROUP
783 ADDITIONAL INFORMATION
784 DEGREE INFORMATION OF CONDUCTIVE HEARING LOSS
903 EXPERT
911 AIR CONDUCTION HEADSET
912 BONE CONDUCTION HEADSET
913 AUDIOMETER
914 RESPONSE BUTTON

**Claims**

1. An information processing device comprising
a prediction unit that predicts a degree of a conductive hearing loss based on test results of an auditory test using air conduction sound, the auditory test being first and second auditory tests including test contents different from each other.

2. The information processing device according to claim 1, wherein

   the test result of the first auditory test includes an air conduction auditory threshold, and
   the test result of the second auditory test includes a test result about an inner ear and/or a posterior labyrinthine.

3. The information processing device according to claim 1, wherein the prediction unit predicts the degree of the conductive hearing loss using a statistical method.

4. The information processing device according to claim 1, wherein the prediction unit predicts the degree of the conductive hearing loss using a learned model.

5. The information processing device according to claim 1, wherein the first auditory test is an air conduction hearing test and/or a self-recording audiometry by intermittent sound.

6. The information processing device according to claim 1, wherein

   the second auditory test
   includes at least one test selected out of a group consisting of a self-recording audiometry by continuous sound, an SISI test, an ABLB test, a DL test, a TD test, a speech recognition threshold test, a highest speech intelligibility test, a distorted speech hearing test, a binaural separation function test, and a sense of direction test.

7. The information processing device according to claim 1, wherein

   the first auditory test is a self-recording audiometry by intermittent sound, and
   the second auditory test is a self-recording audiometry by continuous sound.

8. The information processing device according to claim 1, wherein
the prediction unit predicts the degree of conductive hearing loss based on attribute information of a subject.

9. The information processing device according to claim 1, further comprising
a sound source generation unit that generates the air conduction sound used in the first and second auditory tests.

10. The information processing device according to claim 1, further comprising
a correction unit that acquires output characteristic information of a sound output device, which outputs the generated air conduction sound, and corrects the generated air conduction sound based on the acquired output characteristic information.

11. The information processing device according to claim 10, wherein the correction unit acquires the output characteristic information based on an image of the sound output device.

12. The information processing device according to claim 1, further comprising an output unit that outputs the test result of the first auditory test, the test result of the second auditory test, and the predicted degree of the conductive hearing loss.

13. The information processing device according to claim 1, further comprising an output unit that compares the predicted degree of the conductive hearing loss and a predetermined threshold and outputs, based on a comparison result, information for urging a subject to consult a medical institution.

14. The information processing device according to claim 1, further comprising an output unit that compares the predicted degree of the conductive hearing loss and a predetermined threshold and outputs, based on a comparison result, information for purchasing a hearing aid to a subject.

15. The information processing device according to claim 1, further comprising a parameter determination unit that determines a setting parameter of a hearing aid based on the predicted degree of the conductive hearing loss.

16. The information processing device according to claim 15, wherein the setting parameter is a parameter for performing gain setting control and/or noise suppression setting control for the hearing aid.

17. The information processing device according to claim 1, further comprising a parameter determination unit that determines, based on the predicted degree of the conductive hearing loss, a setting parameter of an acoustic device outside the information processing device.

18. The information processing device according to claim 17, wherein the setting parameter is a parameter for delaying voice output from the acoustic device or controlling volume of the voice output from the acoustic device.

19. An information processing method comprising predicting, by an information processing device, a degree of a conductive hearing loss based on test results of an auditory test using air conduction sound, the auditory test being first and second auditory tests including test contents different from each other.

20. A program for causing a computer to execute a function of predicting a degree of a conductive hearing loss based on test results of an auditory test using air conduction sound, the auditory test being first and second auditory tests including test contents different from each other.

# FIG.1

BONE CONDUCTION SOUND

AIR CONDUCTION SOUND

122

121

OUTER EAR | MIDDLE EAR | INNER EAR | POSTE-RIOR LABY-RINTH

INNER-EAR HEARING LOSS | RETROCOCHLEAR HEARING LOSS

CONDUCTIVE HEARING LOSS | SENSORINEURAL HEARING LOSS

MIXED HEARING LOSS=
CONDUCTIVE HEARING LOSS+
SENSORINEURAL HEARING LOSS

# FIG.2

|  |  |  |  |
|---|---|---|---|
| **CONDUCTIVE HEARING LOSS** | **SENSORINEURAL HEARING LOSS** | | |
|  | | **INNER-EAR HEARING LOSS** | **RETROCOCHLEAR HEARING LOSS** |
| **OUTER EAR/ MIDDLE EAR** | | **INNER EAR** | **POSTERIOR LABYRINTH** |

CORRESPONDING
RANGE

AIR
CONDUCTION
HEARING TEST

CORRESPONDING
RANGE

BONE
CONDUCTION
HEARING TEST

# FIG.3

START

PERFORM AIR
CONDUCTION HEARING
TEST AND BONE
CONDUCTION HEARING
TEST  ~S101

S102

THERE IS
PROBLEM IN
HEARING?

NO

YES

S104

THERE IS
CONDUCTIVE HEARING
LOSS?

NO

YES

S106

TREATMENT
OTHER THAN
HEARING AID IS
NECESSARY?

NO

YES

S103
FOLLOW-UP
OBSERVATION

S107
TREATMENT

S105
STUDY OF HEARING
AID

END

FIG.4

OUTPUT LEVEL [dBSPL]

120 100 80 60 40 20 0

751 752

0 20 40 60 80 100 120
INPUT LEVEL [dBSPL]
EXAMPLE OF CASE OF
CONDUCTIVE HEARING
LOSS

OUTPUT LEVEL [dBSPL]

120 100 80 60 40 20 0

753 754 755

0 20 40 60 80 100 120
INPUT LEVEL [dBSPL]
EXAMPLE OF CASE OF
MIXED HEARING LOSS

OUTPUT LEVEL [dBSPL]

120 100 80 60 40 20 0

756 757 758 759

0 20 40 60 80 100 120
INPUT LEVEL [dBSPL]
EXAMPLE OF CASE OF
SENSORINEURAL
HEARING LOSS

# FIG.5

# FIG.6

116

102

119

117

# FIG.7

# FIG.8

TYPE I

CONTINUOUS SOUND    INTERMITTENT SOUND

1,000Hz(C)     1,000Hz(I)

HEARING LEVEL (dB)

NORMAL
(or CONDUCTIVE HEARING LOSS)

0 2 4 6 8 10 12 (MIN)

TYPE II

CONTINUOUS SOUND    INTERMITTENT SOUND

1,000Hz(C)     1,000Hz(I)

HEARING LEVEL (dB)

INNER-EAR HEARING LOSS
(SENSORINEURAL HEARING LOSS)

0 2 4 6 8 10 12 (MIN)

TYPE III

CONTINUOUS SOUND    INTERMITTENT SOUND

1,000Hz(C)     1,000Hz(I)

HEARING LEVEL (dB)

RETROCOCHLEAR HEARING LOSS
(SENSORINEURAL HEARING LOSS)

0 2 4 6 8 10 12 (MIN)

TYPE IV

CONTINUOUS SOUND    INTERMITTENT SOUND

1,000Hz(C)     1,000Hz(I)

HEARING LEVEL (dB)

RETROCOCHLEAR HEARING LOSS
(SENSORINEURAL HEARING LOSS)

0 2 4 6 8 10 12 (MIN)

TYPE V

CONTINUOUS SOUND    INTERMITTENT SOUND

1,000Hz(C)     1,000Hz(I)

HEARING LEVEL (dB)

FUNCTIONAL HEARING LOSS

0 2 4 6 8 10 12 (MIN)

# FIG.9

| CONDUCTIVE HEARING LOSS | SENSORINEURAL HEARING LOSS | |
|---|---|---|
| | INNER-EAR HEARING LOSS | RETROCOCHLEAR HEARING LOSS |
| OUTER EAR/MIDDLE EAR | INNER EAR | POSTERIOR LABYRINTH |

SELF-RECORDING AUDIOMETRY (INTERMITTENT SOUND)

333    334    335

SELF-RECORDING AUDIOMETRY (CONTINUOUS SOUND)

336    337    338

# FIG.10

| CONDUCTIVE HEARING LOSS | SENSORINEURAL HEARING LOSS | |
|---|---|---|
| | INNER-EAR HEARING LOSS | RETROCOCHLEAR HEARING LOSS |
| OUTER EAR/MIDDLE EAR | INNER EAR | POSTERIOR LABYRINTH |

AUDITORY TEST OF FIRST GROUP

541

AUDITORY TEST OF SECOND GROUP

542    543    544

545    546

# FIG.11

| NUMBER | AIR CONDUC-TION HEARING LEVEL | FIRST TEST | SECOND TEST | THIRD TEST | ... | M-TH TEST | ADDI-TIONAL INFORMA-TION | CONDUC-TIVE HEARING LOSS SCORE |
|---|---|---|---|---|---|---|---|---|
| 1 | 60 | + | + | - | ... | - | 0.1 | 0.1 |
| 2 | 55 | + | + | + | ... | + | 0.2 | 0.1 |
| 3 | 40 | - | - | - | ... | - | 0.8 | 1.0 |
| 4 | 45 | + | - | + | ... | + | 0.6 | 0.2 |
| ⋮ | ⋮ | ⋮ | ⋮ | ⋮ | ⋱ | ⋮ | ⋮ | ⋮ |
| N-1 | 35 | - | + | + | ... | + | 0.1 | 0.0 |
| N | 50 | - | - | - | ... | + | 0.3 | 0.6 |

781 782 783 784

# FIG.12

EP 4 603 015 A1

# FIG.13

AUDITORY TEST INFORMATION
OF FIRST GROUP →

AUDITORY TEST INFORMATION
OF SECOND GROUP →

ADDITIONAL INFORMATION →

164

CONDUCTIVE
HEARING LOSS
DEGREE
PREDICTION UNIT

→ DEGREE
PREDICTION
INFORMATION OF
CONDUCTIVE
HEARING LOSS

# FIG.14

START

CARRY OUT AUDITORY TEST OF FIRST
GROUP AND AUDITORY TEST OF SECOND
GROUP

— S201

PREDICT DEGREE OF CONDUCTIVE
HEARING LOSS

— S202

END

# FIG.15

START

CARRY OUT AUDITORY TEST OF FIRST GROUP — S301

THERE IS PROBLEM IN AIR CONDUCTION HEARING LEVEL? — S302

NO

YES

CARRY OUT AUDITORY TEST OF SECOND GROUP — S303

PREDICT DEGREE OF CONDUCTIVE HEARING LOSS — S304

END

# FIG.16

EP 4 603 015 A1

FIG.17

# FIG.18

# FIG.19

```
        ┌─────────────┐
        │    START    │
        └──────┬──────┘
               ▼
┌─────────────────────────────┐
│    SET LEVEL/FREQUENCY       │
│ CHARACTERISTIC CORRECTION    │──S401
│        INFORMATION           │
└──────────────┬──────────────┘
               ▼
┌─────────────────────────────┐
│  CARRY OUT AUDITORY TEST OF FIRST │
│ GROUP AND AUDITORY TEST OF SECOND │──S402
│            GROUP             │
└──────────────┬──────────────┘
               ▼
┌─────────────────────────────┐
│  PREDICT DEGREE OF CONDUCTIVE │──S403
│       HEARING LOSS           │
└──────────────┬──────────────┘
               ▼
        ┌─────────────┐
        │     END     │
        └─────────────┘
```

# FIG.20

```
                    ┌──────────────┐
                    │    START     │
                    └──────┬───────┘
                           │
                           ▼
          ┌─────────────────────────────┐
          │ CARRY OUT AUDITORY TEST      │
          │ OF FIRST GROUP AND           │──S501
          │ AUDITORY TEST OF SECOND      │
          │ GROUP                        │
          └──────────────┬──────────────┘
                         │
                         ▼
          ┌─────────────────────────────┐
          │ PREDICT DEGREE OF            │──S502
          │ CONDUCTIVE HEARING LOSS      │
          └──────────────┬──────────────┘
                         │
                         ▼
                                          S503
                  ◇ THERE IS PROBLEM IN ◇
        NO ◄──────── HEARING? ────────
                         │ YES
                         ▼
                                          S505
                  ◇ DEGREE             ◇
                    PREDICTION VALUE
                    OF CONDUCTIVE        ──── NO
                    HEARING LOSS IS
                    HIGH?
                         │ YES
                         ▼
                                          S507
                  ◇ HAS                ◇
                    NOT ALREADY
                    CONFIRMED WITH
                    MEDICAL PRACTITIONER  ──── NO
                    OR THE LIKE THAT THERE
                    IS NO PROBLEM IN
                    HEARING AID
                    USE?
                         │ YES
```

| S504 | S508 | S506 |
|---|---|---|
| RECOMMEND FOLLOW-UP OBSERVATION | RECOMMEND VISIT TO MEDICAL INSTITUTION | STUDY OF HEARING AID |

```
                    ┌──────────────┐
                    │     END      │
                    └──────────────┘
```

# FIG.21

```
                    ┌──────────────┐
                    │    START     │
                    └──────┬───────┘
                           ↓
            ┌────────────────────────────────┐
            │ PERFORM AUDITORY TEST OF FIRST │  ~S601
            │            GROUP               │
            └───────────────┬────────────────┘
                           ↓
            ┌────────────────────────────────┐
            │            i←0                 │  ~S602
            └───────────────┬────────────────┘
                           ↓
            ┌────────────────────────────────┐
            │            i←i+1               │  ~S603
            └───────────────┬────────────────┘
                           ↓
            ┌────────────────────────────────┐
            │  PERFORM i-TH TEST OF AUDITORY │  ~S604
            │      TEST OF SECOND GROUP      │
            └───────────────┬────────────────┘
                           ↓
            ┌────────────────────────────────┐
            │  PREDICT DEGREE OF CONDUCTIVE  │
            │ HEARING LOSS AND PROBABILITY OF│  ~S605
            │ DEGREE OF CONDUCTIVE HEARING   │
            │            LOSS                │
            └───────────────┬────────────────┘
                           ↓
                        S606
                      ◇────────◇
                     ╱  i≠M?    ╲      NO
                     ╲          ╱───────────┐
                      ◇────────◇            │
                        │ YES               │
                        ↓                   │
                      S607                  │
           NO       ◇──────────◇            │
        ┌───────────╱ PROBABILITY ╲         │
        │           ╲ IS SUFFICIENT?╱        │
        │            ◇──────────◇           │
        │              │ YES                │
        │              ↓                    │
        │            ( 1 )←─────────────────┘
        └──→
```

$i \leftarrow 0$

$i \leftarrow i+1$

$i \neq M$?

# FIG.22

① → S608 THERE IS PROBLEM IN HEARING?

NO → S609 RECOMMEND FOLLOW-UP OBSERVATION

YES → S610 DEGREE PREDICTION VALUE OF CONDUCTIVE HEARING LOSS IS HIGH?

NO → S611 STUDY OF HEARING AID

YES → S612 HAS NOT ALREADY CONFIRMED WITH MEDICAL PRACTITIONER OR THE LIKE THAT THERE IS NO PROBLEM IN HEARING AID USE?

NO

YES → S613 RECOMMEND VISIT TO MEDICAL INSTITUTION

END

# FIG.23

START

CARRY OUT AUDITORY TEST OF FIRST GROUP AND AUDITORY TEST OF SECOND GROUP — S701

PREDICT DEGREE OF CONDUCTIVE HEARING LOSS — S702

S703 THERE IS PROBLEM IN HEARING? — NO / YES

S705 DEGREE PREDICTION VALUE OF CONDUCTIVE HEARING LOSS IS HIGH? — NO / YES

S707 HAS NOT ALREADY CONFIRMED WITH MEDICAL PRACTITIONER OR THE LIKE THAT THERE IS NO PROBLEM IN HEARING AID USE? — NO / YES

S704 RECOMMEND FOLLOW-UP OBSERVATION

S708 RECOMMEND VISIT TO MEDICAL INSTITUTION

S706 TRANSITION TO HEARING AID PURCHASE SCREEN

END

# FIG.24

EP 4 603 015 A1

# FIG.25

```
      ┌──────────────┐
      │    START     │
      └──────┬───────┘
             │
             ▼
┌────────────────────────────────┐
│ CARRY OUT AUDITORY TEST OF FIRST│
│ GROUP AND AUDITORY TEST OF SECOND├── S801
│             GROUP               │
└──────────────┬─────────────────┘
               │
               ▼
┌────────────────────────────────┐
│   PREDICT DEGREE OF CONDUCTIVE  │
│ HEARING LOSS AND BONE CONDUCTION ├── S802
│         HEARING LEVEL           │
└──────────────┬─────────────────┘
               │
               ▼
┌────────────────────────────────┐
│  DETERMINE SETTING OF HEARING AID│
│  USING PREDICTION VALUES OF AIR │
│ CONDUCTION HEARING LEVEL AND BONE├── S803
│    CONDUCTION HEARING LEVEL     │
└──────────────┬─────────────────┘
               │
               ▼
      ┌──────────────┐
      │     END      │
      └──────────────┘
```

# FIG.26

# FIG.27

FREQUENCY [Hz]

BONE CONDUCTION:PREDICTION VALUE

# FIG.28

| | 125Hz | 250Hz | 500Hz | 1000Hz | 2000Hz | 4000Hz | 8000Hz | PREDICTION FLAG |
|---|---|---|---|---|---|---|---|---|
| AIR CONDUCTION | 55 | 55 | 60 | 60 | 65 | 70 | 65 | |
| BONE CONDUCTION | | 30 | 30 | 30 | 35 | 35 | | 1 |

EP 4 603 015 A1

# FIG.29

120

TV

MESSAGE

YES

NO

116

117

# FIG.30

Omsec          20msec          40msec

Omsec          20msec          40msec

Omsec          20msec          40msec

# FIG.31

# FIG.32

# FIG.33

# FIG.34

INFORMATION PROCESSING TERMINAL — 40

INPUT UNIT — 41

CONTROL UNIT — 46

OUTPUT UNIT — 43

DISPLAY UNIT — 44

COMMUNICA-TION UNIT — 42

STORAGE UNIT — 45

PROGRAM — 451

# FIG.35

SERVER — 90

COMMUNICA-TION UNIT — 91

CONTROL UNIT — 96

STORAGE UNIT — 95

PROGRAM — 961

# FIG.36

# FIG.37

| DATA THAT CAN BE OBTAINED IN EDGE REGION | DEVICE DATA |
| --- | --- |
| | USE HISTORY DATA |
| | PERSONALIZED DATA |
| | BIOLOGICAL DATA |
| | EMOTIONAL DATA |
| | APPLICATION DATA |
| | FITTING DATA |
| | PREFERENCE DATA |
| | ... |

# FIG.38

# FIG.39

| Headphones/TWS | OTC-Hearing Aid | HEARING AID |
|:---:|:---:|:---:|
| NORMAL LISTENER | LIGHT/MODERATE HEARING-IMPAIRED PERSON | SEVERE HEARING-IMPAIRED PERSON |

EP 4 603 015 A1

## INTERNATIONAL SEARCH REPORT

| International application No. |
|---|
| **PCT/JP2023/033500** |

**A. CLASSIFICATION OF SUBJECT MATTER**

*A61B 5/12*(2006.01)i
FI: A61B5/12

According to International Patent Classification (IPC) or to both national classification and IPC

**B. FIELDS SEARCHED**

Minimum documentation searched (classification system followed by classification symbols)

A61B5/06 - 5/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2010-518884 A (UMEDICAL CO., LTD.) 03 June 2010 (2010-06-03) paragraphs [0010]-[0028], fig. 1 | 1-10, 12-20 |
| A | paragraphs [0010]-[0028], fig. 1 | 11 |
| Y | US 2007/0258609 A1 (SIEMENS AUDIOLOGISCHE TECHNIK GMBH) 08 November 2007 (2007-11-08) paragraphs [0002]-[0003], [0009] | 1-10, 12-20 |
| A | paragraphs [0002]-[0003], [0009] | 11 |
| Y | JP 7-143976 A (RION CO LTD) 06 June 1995 (1995-06-06) paragraphs [0060]-[0066] | 6-7 |
| Y | JP 2013-75066 A (TOSHIBA CORP) 25 April 2013 (2013-04-25) paragraphs [0020]-[0023], [0038], fig. 3-4 | 8-10 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **10 November 2023** | **21 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/033500**

| Patent document cited in search report | | | Publication date (day/month/year) | Patent family member(s) | | | Publication date (day/month/year) |
|---|---|---|---|---|---|---|---|
| JP | 2010-518884 | A | 03 June 2010 | US | 2009/0288489 | A1 | |
| | | | | paragraphs [0014]-[0039], fig. 1 | | | |
| | | | | WO | 2008/032927 | A1 | |
| | | | | KR | 10-2008-0024611 | A | |
| US | 2007/0258609 | A1 | 08 November 2007 | EP | 1853090 | A2 | |
| | | | | DE | 102006020833 | B3 | |
| JP | 7-143976 | A | 06 June 1995 | (Family: none) | | | |
| JP | 2013-75066 | A | 25 April 2013 | US | 2013/0083930 | A1 | |
| | | | | paragraphs [0026]-[0031], [0045]-[0046], fig. 3-4 | | | |

Form PCT/ISA/210 (patent family annex) (January 2015)

# EP 4 603 015 A1

**REFERENCES CITED IN THE DESCRIPTION**

**Non-patent literature cited in the description**

- Hearing Test Method of Japanese Society of Hearing Medicine 1. *Pure Sound Hearing (Threshold) Level Measurement Method by Audiometer*, 2008